# EUROPEAN PATENT SPECIFICATION

(11) **EP 1 654 282 B1**
(45) Date of publication and mention of the grant of the patent: **01.12.2010**
(21) Application number: 04779661.0
(22) Date of filing: 30.07.2004
(51) Int. Cl.: C07K 14/78, A61K 9/48

(54) **GELATIN CAPSULES**
GELATINKAPSELN
GELULES DE GELATINE

(30) Priority: 01.08.2003 US 492085 P; 29.07.2004 US 901816
(43) Date of publication of application: 10.05.2006
(73) Proprietor: FIBROGEN, INC., South San Francisco, CA 94080 (US)
(72) Inventor: CHANG, Robert, C., Burlingame, CA 94010 (US); OLSEN, David, R., Menlo Park, CA 94025 (US); POLAREK, James, W., Sausalito, CA 94965 (US); WILLIAMS, Kim, E., Menlo Park, CA 94025 (US)
(74) Representative: Goodfellow, Hugh Robin
(86) International application number: PCT/US2004/024663
(87) International publication number: WO 2005/012356

(56) References cited:
- WO-A-01/34646
- WERTEN MARC W T ET AL: "Secreted production of a custom-designed, highly hydrophilic gelatin in Pichia pastoris" PROTEIN ENGINEERING, vol. 14, no. 6, June 2001 (2001-06), pages 447-454, XP002302730 ISSN: 0269-2139
- DATABASE GENESEQ 25 July 2000 (2000-07-25), "A human collagen 1 (alpha1) protein helical region" XP002302731 Database accession no. AAY84544
- DATABASE GENESEQ 12 July 2000 (2000-07-12), "Amino acid sequence of human type 1 (alpha1) collagen polypeptide" XP002302732 Database accession no. AAY84403
- DATABASE GENESEQ 25 July 2000 (2000-07-25), "Aminoa cid sequence of a human collagen 1 (alpha1) protein" XP002302733 Database accession no. AAY84541
- DATABASE EPO PROTEINS 7 May 1999 (1999-05-07), "Sequence 1 from Patent EP0829724" XP002302734 Database accession no. A70002

### SEQUENCE LISTING

<110> FibroGen, Inc.
   Chang, Robert C.
   Olsen, David R.
   James, Polarek w.
   Williams, Kim E.
<120> Gelatin Capsules
<130> FP0404 PCT
<150> US 60/492,085
   <151> 2003-08-01
<160> 13
<170> PatentIn version 3.2
<210> 1
   <211> 498
   <212> PRT
   <213> Homo sapiens
<400> 1
   Gly Leu Pro Gly Ala Lys Gly Leu Thr Gly Ser Pro Gly Ser Pro Gly
   1               5                   10                  15
   Pro Asp Gly Lys Thr Gly Pro Pro Gly Pro Ala Gly Gln Asp Gly Arg
               20                  25                  30
   Pro Gly Pro Pro Gly Pro Pro Gly Ala Arg Gly Gln Ala Gly Val Met
           35                  40                  45
   Gly Phe Pro Gly Pro Lys Gly Ala Ala Gly Glu Pro Gly Lys Ala Gly
       50                  55                  60
   Glu Arg Gly Val Pro Gly Pro Pro Gly Ala Val Gly Pro Ala Gly Lys
   65                  70                  75                  80
   Asp Gly Glu Ala Gly Ala Gln Gly Pro Pro Gly Pro Ala Gly Pro Ala
                   85                  90                  95
   Gly Glu Arg Gly Glu Gln Gly Pro Ala Gly Ser Pro Gly Phe Gln Gly
               100                 105                 110
   Leu Pro Gly Pro Ala Gly Pro Pro Gly Glu Ala Gly Lys Pro Gly Glu
           115                 120                 125
   Gln Gly Val Pro Gly Asp Leu Gly Ala Pro Gly Pro Ser Gly Ala Arg
       130                 135                 140
   Gly Glu Arg Gly Phe Pro Gly Glu Arg Gly Val Gln Gly Pro Pro Gly
   145                 150                 155                 160
   Pro Ala Gly Pro Arg Gly Ala Asn Gly Ala Pro Gly Asn Asp Gly Ala
                   165                 170                 175
   Lys Gly Asp Ala Gly Ala Pro Gly Ala Pro Gly Ser Gln Gly Ala Pro
               180                 185                 190
   Gly Leu Gln Gly Met Pro Gly Glu Arg Gly Ala Ala Gly Leu Pro Gly
           195                 200                 205
   Pro Lys Gly Asp Arg Gly Asp Ala Gly Pro Lys Gly Ala Asp Gly Ser
       210                 215                 220
   Pro Gly Lys Asp Gly Val Arg Gly Leu Thr Gly Pro Ile Gly Pro Pro
   225                 230                 235                 240
   Gly Pro Ala Gly Ala Pro Gly Asp Lys Gly Glu Ser Gly Pro Ser Gly
                   245                 250                 255
   Pro Ala Gly Pro Thr Gly Ala Arg Gly Ala Pro Gly Asp Arg Gly Glu
               260                 265                 270
   Pro Gly Pro Pro Gly Pro Ala Gly Phe Ala Gly Pro Pro Gly Ala Asp
           275                 280                 285
   Gly Gln Pro Gly Ala Lys Gly Glu Pro Gly Asp Ala Gly Ala Lys Gly
       290                 295                 300
   Asp Ala Gly Pro Pro Gly Pro Ala Gly Pro Ala Gly Pro Pro Gly Pro
   305                 310                 315                 320
   Ile Gly Asn Val Gly Ala Pro Gly Ala Lys Gly Ala Arg Gly Ser Ala
                   325                 330                 335
   Gly Pro Pro Gly Ala Thr Gly Phe Pro Gly Ala Ala Gly Arg Val Gly
               340                 345                 350
   Pro Pro Gly Pro Ser Gly Asn Ala Gly Pro Pro Gly Pro Pro Gly Pro
           355                 360                 365
   Ala Gly Lys Glu Gly Gly Lys Gly Pro Arg Gly Glu Thr Gly Pro Ala
       370                 375                 380
   Gly Arg Pro Gly Glu Val Gly Pro Pro Gly Pro Pro Gly Pro Ala Gly
   385                 390                 395                 400
   Glu Lys Gly Ser Pro Gly Ala Asp Gly Pro Ala Gly Ala Pro Gly Thr
                   405                 410                 415
   Pro Gly Pro Gln Gly Ile Ala Gly Gln Arg Gly Val Val Gly Leu Pro
               420                 425                 430
   Gly Gln Arg Gly Glu Arg Gly Phe Pro Gly Leu Pro Gly Pro Ser Gly
           435                 440                 445
   Glu Pro Gly Lys Gln Gly Pro Ser Gly Ala Ser Gly Glu Arg Gly Pro
   450 455 460
   Pro Gly Pro Met Gly Pro Pro Gly Leu Ala Gly Pro Pro Gly Glu Ser
   465 470 475 480
   Gly Arg Glu Gly Ala Pro Gly Ala Glu Gly Ser Pro Gly Arg Asp Gly
   485 490 495
   Ser Pro
<210> 2
   <211> 498
   <212> PRT
   <213> Homo sapiens
<400> 2
   Gly Leu Pro Gly Ala Lys Gly Leu Thr Gly Ser Pro Gly Ser Pro Gly
   1               5                   10                  15
   Pro Asp Gly Lys Thr Gly Pro Pro Gly Pro Ala Gly Gln Asp Gly Arg
               20                  25                  30
   Pro Gly Pro Pro Gly Pro Pro Gly Ala Arg Gly Gln Ala Gly Pro Pro
           35                  40                  45
   Gly Phe Pro Gly Pro Lys Gly Ala Ala Gly Glu Pro Gly Lys Ala Gly
       50                  55                  60
   Glu Arg Gly Val Pro Gly Pro Pro Gly Ala Val Gly Pro Ala Gly Lys
   65                  70                  75                  80
   Asp Gly Glu Ala Gly Ala Gln Gly Pro Pro Gly Pro Ala Gly Pro Ala
                   85                  90                  95
   Gly Glu Arg Gly Glu Gln Gly Pro Ala Gly Ser Pro Gly Phe Gln Gly
               100                 105                 110
   Leu Pro Gly Pro Ala Gly Pro Pro Gly Glu Ala Gly Lys Pro Gly Glu
           115                 120                 125
   Gln Gly Val Pro Gly Asp Leu Gly Ala Pro Gly Pro Ser Gly Ala Arg
       130                 135                 140
   Gly Glu Arg Gly Phe Pro Gly Glu Arg Gly Val Gln Gly Pro Pro Gly
   145                 150                 155                 160
   Pro Ala Gly Pro Arg Gly Ala Asn Gly Ala Pro Gly Asn Asp Gly Ala
                   165                 170                 175
   Lys Gly Asp Ala Gly Ala Pro Gly Ala Pro Gly Ser Gln Gly Ala Pro
   180 185 190
   Gly Leu Gln Gly Met Pro Gly Glu Arg Gly Ala Ala Gly Leu Pro Gly
   195 200 205
   Pro Lys Gly Asp Arg Gly Asp Ala Gly Pro Lys Gly Ala Asp Gly Ser
   210 215 220
   Pro Gly Lys Asp Gly Val Arg Gly Leu Thr Gly Pro Ile Gly Pro Pro
   225 230 235 240
   Gly Pro Ala Gly Ala Pro Gly Asp Lys Gly Glu Ser Gly Pro Ser Gly
   245 250 255
   Pro Ala Gly Pro Thr Gly Ala Arg Gly Ala Pro Gly Asp Arg Gly Glu
   260 265 270
   Pro Gly Pro Pro Gly Pro Ala Gly Phe Ala Gly Pro Pro Gly Ala Asp
   275 280 285
   Gly Gln Pro Gly Ala Lys Gly Glu Pro Gly Asp Ala Gly Ala Lys Gly
   290 295 300
   Asp Ala Gly Pro Pro Gly Pro Ala Gly Pro Ala Gly Pro Pro Gly Pro
   305 310 315 320
   Ile Gly Asn Val Gly Ala Pro Gly Ala Lys Gly Ala Arg Gly Ser Ala
   325 330 335
   Gly Pro Pro Gly Ala Thr Gly Phe Pro Gly Ala Ala Gly Arg Val Gly
   340 345 350
   Pro Pro Gly Pro Ser Gly Asn Ala Gly Pro Pro Gly Pro Pro Gly Pro
   355 360 365
   Ala Gly Lys Glu Gly Gly Lys Gly Pro Arg Gly Glu Thr Gly Pro Ala
   370 375 380
   Gly Arg Pro Gly Glu Val Gly Pro Pro Gly Pro Pro Gly Pro Ala Gly
   385 390 395 400
   Glu Lys Gly Ser Pro Gly Ala Asp Gly Pro Ala Gly Ala Pro Gly Thr
   405 410 415
   Pro Gly Pro Gln Gly Ile Ala Gly Gln Arg Gly Val Val Gly Leu Pro
   420 425 430
   Gly Gln Arg Gly Glu Arg Gly Phe Pro Gly Leu Pro Gly Pro Ser Gly
   435 440 445
   Glu Pro Gly Lys Gln Gly Pro Ser Gly Ala Ser Gly Glu Arg Gly Pro
   450 455 460
   Pro Gly Pro Met Gly Pro Pro Gly Leu Ala Gly Pro Pro Gly Glu Ser
   465 470 475 480
   Gly Arg Glu Gly Ala Pro Gly Ala Glu Gly Ser Pro Gly Arg Asp Gly
   485 490 495
   Ser Pro
<210> 3
   <211> 660
   <212> PRT
   <213> Homo sapiens
<400> 3
   Gly Leu Pro Gly Ala Lys Gly Leu Thr Gly Ser Pro Gly Ser Pro Gly
   1               5                   10                  15
   Pro Asp Gly Lys Thr Gly Pro Pro Gly Pro Ala Gly Gln Asp Gly Arg
               20                  25                  30
   Pro Gly Pro Pro Gly Pro Pro Gly Ala Arg Gly Gln Ala Gly Val Met
           35                  40                  45
   Gly Phe Pro Gly Pro Lys Gly Ala Ala Gly Glu Pro Gly Lys Ala Gly
       50                  55                  60
   Glu Arg Gly Val Pro Gly Pro Pro Gly Ala Val Gly Pro Ala Gly Lys
   65                  70                  75                  80
   Asp Gly Glu Ala Gly Ala Gln Gly Pro Pro Gly Pro Ala Gly Pro Ala
                   85                  90                  95
   Gly Glu Arg Gly Glu Gln Gly Pro Ala Gly Ser Pro Gly Phe Gln Gly
               100                 105                 110
   Leu Pro Gly Pro Ala Gly Pro Pro Gly Glu Ala Gly Lys Pro Gly Glu
           115                 120                 125
   Gln Gly Val Pro Gly Asp Leu Gly Ala Pro Gly Pro Ser Gly Ala Arg
       130                 135                 140
   Gly Glu Arg Gly Phe Pro Gly Glu Arg Gly Val Gln Gly Pro Pro Gly
   145                 150                 155                 160
   Pro Ala Gly Pro Arg Gly Ala Asn Gly Ala Pro Gly Asn Asp Gly Ala
                   165                 170                 175
   Lys Gly Asp Ala Gly Ala Pro Gly Ala Pro Gly Ser Gln Gly Ala Pro
   180 185 190
   Gly Leu Gln Gly Met Pro Gly Glu Arg Gly Ala Ala Gly Leu Pro Gly
   195 200 205
   Pro Lys Gly Asp Arg Gly Asp Ala Gly Pro Lys Gly Ala Asp Gly Ser
   210 215 220
   Pro Gly Lys Asp Gly Val Arg Gly Leu Thr Gly Pro Ile Gly Pro Pro
   225 230 235 240
   Gly Pro Ala Gly Ala Pro Gly Asp Lys Gly Glu Ser Gly Pro Ser Gly
   245 250 255
   Pro Ala Gly Pro Thr Gly Ala Arg Gly Ala Pro Gly Asp Arg Gly Glu
   260 265 270
   Pro Gly Pro Pro Gly Pro Ala Gly Phe Ala Gly Pro Pro Gly Ala Asp
   275 280 285
   Gly Gln Pro Gly Ala Lys Gly Glu Pro Gly Asp Ala Gly Ala Lys Gly
   290 295 300
   Asp Ala Gly Pro Pro Gly Pro Ala Gly Pro Ala Gly Pro Pro Gly Pro
   305 310 315 320
   Ile Gly Asn Val Gly Ala Pro Gly Ala Lys Gly Ala Arg Gly Ser Ala
   325 330 335
   Gly Pro Pro Gly Ala Thr Gly Phe Pro Gly Ala Ala Gly Arg Val Gly
   340 345 350
   Pro Pro Gly Pro Ser Gly Asn Ala Gly Pro Pro Gly Pro Pro Gly Pro
   355 360 365
   Ala Gly Lys Glu Gly Gly Lys Gly Pro Arg Gly Glu Thr Gly Pro Ala
   370 375 380
   Gly Arg Pro Gly Glu Val Gly Pro Pro Gly Pro Pro Gly Pro Ala Gly
   385 390 395 400
   Glu Lys Gly Ser Pro Gly Ala Asp Gly Pro Ala Gly Ala Pro Gly Thr
   405 410 415
   Pro Gly Pro Gln Gly Ile Ala Gly Gln Arg Gly Val Val Gly Leu Pro
   420 425 430
   Gly Gln Arg Gly Glu Arg Gly Phe Pro Gly Leu Pro Gly Pro Ser Gly
   435 440 445
   Glu Pro Gly Lys Gln Gly Pro Ser Gly Ala Ser Gly Glu Arg Gly Pro
       450                 455                 460
   Pro Gly Pro Met Gly Pro Pro Gly Leu Ala Gly Pro Pro Gly Glu Ser
   465                 470                 475                 480
   Gly Arg Glu Gly Ala Pro Gly Ala Glu Gly Ser Pro Gly Arg Asp Gly
                   485                 490                 495
   Ser Pro Gly Ala Lys Gly Asp Arg Gly Glu Thr Gly Pro Ala Gly Pro
               500                 505                 510
   Pro Gly Ala Pro Gly Ala Pro Gly Ala Pro Gly Pro Val Gly Pro Ala
           515                 520                 525
   Gly Lys Ser Gly Asp Arg Gly Glu Thr Gly Pro Ala Gly Pro Ala Gly
       530                 535                 540
   Pro Val Gly Pro Ala Gly Ala Arg Gly Pro Ala Gly Pro Gln Gly Pro
   545                 550                 555                 560
   Arg Gly Asp Lys Gly Glu Thr Gly Glu Gln Gly Asp Arg Gly Ile Lys
                   565                 570                 575
   Gly His Arg Gly Phe Ser Gly Leu Gln Gly Pro Pro Gly Pro Pro Gly
               580                 585                 590
   Ser Pro Gly Glu Gln Gly Pro Ser Gly Ala Ser Gly Pro Ala Gly Pro
           595                 600                 605
   Arg Gly Pro Pro Gly Ser Ala Gly Ala Pro Gly Lys Asp Gly Leu Asn
       610                 615                 620
   Gly Leu Pro Gly Pro Ile Gly Pro Pro Gly Pro Arg Gly Arg Thr Gly
   625                 630                 635                 640
   Asp Ala Gly Pro Val Gly Pro Pro Gly Pro Pro Gly Pro Pro Gly Pro
                   645                 650                 655
   Pro Gly Pro Pro
               660
<210> 4
   <211> 660
   <212> PRT
   <213> Homo sapiens
<400> 4
   Gly Leu Pro Gly Ala Lys Gly Leu Thr Gly Ser Pro Gly Ser Pro Gly
   1               5                   10                  15
   Pro Asp Gly Lys Thr Gly Pro Pro Gly Pro Ala Gly Gln Asp Gly Arg
   20 25 30
   Pro Gly Pro Pro Gly Pro Pro Gly Ala Arg Gly Gln Ala Gly Pro Pro
   35 40 45
   Gly Phe Pro Gly Pro Lys Gly Ala Ala Gly Glu Pro Gly Lys Ala Gly
   50 55 60
   Glu Arg Gly Val Pro Gly Pro Pro Gly Ala Val Gly Pro Ala Gly Lys
   65 70 75 80
   Asp Gly Glu Ala Gly Ala Gln Gly Pro Pro Gly Pro Ala Gly Pro Ala
   85 90 95
   Gly Glu Arg Gly Glu Gln Gly Pro Ala Gly Ser Pro Gly Phe Gln Gly
   100 105 110
   Leu Pro Gly Pro Ala Gly Pro Pro Gly Glu Ala Gly Lys Pro Gly Glu
   115 120 125
   Gln Gly Val Pro Gly Asp Leu Gly Ala Pro Gly Pro Ser Gly Ala Arg
   130 135 140
   Gly Glu Arg Gly Phe Pro Gly Glu Arg Gly Val Gln Gly Pro Pro Gly
   145 150 155 160
   Pro Ala Gly Pro Arg Gly Ala Asn Gly Ala Pro Gly Asn Asp Gly Ala
   165 170 175
   Lys Gly Asp Ala Gly Ala Pro Gly Ala Pro Gly Ser Gln Gly Ala Pro
   180 185 190
   Gly Leu Gln Gly Met Pro Gly Glu Arg Gly Ala Ala Gly Leu Pro Gly
   195 200 205
   Pro Lys Gly Asp Arg Gly Asp Ala Gly Pro Lys Gly Ala Asp Gly Ser
   210 215 220
   Pro Gly Lys Asp Gly Val Arg Gly Leu Thr Gly Pro Ile Gly Pro Pro
   225 230 235 240
   Gly Pro Ala Gly Ala Pro Gly Asp Lys Gly Glu Ser Gly Pro Ser Gly
   245 250 255
   Pro Ala Gly Pro Thr Gly Ala Arg Gly Ala Pro Gly Asp Arg Gly Glu
   260 265 270
   Pro Gly Pro Pro Gly Pro Ala Gly Phe Ala Gly Pro Pro Gly Ala Asp
   275 280 285
   Gly Gln Pro Gly Ala Lys Gly Glu Pro Gly Asp Ala Gly Ala Lys Gly
   290 295 300
   Asp Ala Gly Pro Pro Gly Pro Ala Gly Pro Ala Gly Pro Pro Gly Pro
   305 310 315 320
   Ile Gly Asn Val Gly Ala Pro Gly Ala Lys Gly Ala Arg Gly Ser Ala
   325 330 335
   Gly Pro Pro Gly Ala Thr Gly Phe Pro Gly Ala Ala Gly Arg Val Gly
   340 345 350
   Pro Pro Gly Pro Ser Gly Asn Ala Gly Pro Pro Gly Pro Pro Gly Pro
   355 360 365
   Ala Gly Lys Glu Gly Gly Lys Gly Pro Arg Gly Glu Thr Gly Pro Ala
   370 375 380
   Gly Arg Pro Gly Glu Val Gly Pro Pro Gly Pro Pro Gly Pro Ala Gly
   385 390 395 400
   Glu Lys Gly Ser Pro Gly Ala Asp Gly Pro Ala Gly Ala Pro Gly Thr
   405 410 415
   Pro Gly Pro Gln Gly Ile Ala Gly Gln Arg Gly Val Val Gly Leu Pro
   420 425 430
   Gly Gln Arg Gly Glu Arg Gly Phe Pro Gly Leu Pro Gly Pro Ser Gly
   435 440 445
   Glu Pro Gly Lys Gln Gly Pro Ser Gly Ala Ser Gly Glu Arg Gly Pro
   450 455 460
   Pro Gly Pro Met Gly Pro Pro Gly Leu Ala Gly Pro Pro Gly Glu Ser
   465 470 475 480
   Gly Arg Glu Gly Ala Pro Gly Ala Glu Gly Ser Pro Gly Arg Asp Gly
   485 490 495
   Ser Pro Gly Ala Lys Gly Asp Arg Gly Glu Thr Gly Pro Ala Gly Pro
   500 505 510
   Pro Gly Ala Pro Gly Ala Pro Gly Ala Pro Gly Pro Val Gly Pro Ala
   515 520 525
   Gly Lys Ser Gly Asp Arg Gly Glu Thr Gly Pro Ala Gly Pro Ala Gly
   530 535 540
   Pro Val Gly Pro Ala Gly Ala Arg Gly Pro Ala Gly Pro Gln Gly Pro
   545 550 555 560
   Arg Gly Asp Lys Gly Glu Thr Gly Glu Gln Gly Asp Arg Gly Ile Lys
   565 570 575
   Gly His Arg Gly Phe Ser Gly Leu Gln Gly Pro Pro Gly Pro Pro Gly
   580 585 590
   Ser Pro Gly Glu Gln Gly Pro Ser Gly Ala Ser Gly Pro Ala Gly Pro
   595 600 605
   Arg Gly Pro Pro Gly Ser Ala Gly Ala Pro Gly Lys Asp Gly Leu Asn
   610 615 620
   Gly Leu Pro Gly Pro Ile Gly Pro Pro Gly Pro Arg Gly Arg Thr Gly
   625 630 635 640
   Asp Ala Gly Pro Val Gly Pro Pro Gly Pro Pro Gly Pro Pro Gly Pro
   645 650 655
   Pro Gly Pro Pro
   660
<210> 5
   <211> 1014
   <212> PRT
   <213> Homo sapiens
<400> 5
   Gly Pro Met Gly Pro Ser Gly Pro Arg Gly Leu Pro Gly Pro Pro Gly
   1               5                   10                  15
   Ala Pro Gly Pro Gln Gly Phe Gln Gly Pro Pro Gly Glu Pro Gly Glu
               20                  25                  30
   Pro Gly Ala Ser Gly Pro Met Gly Pro Arg Gly Pro Pro Gly Pro Pro
           35                  40                  45
   Gly Lys Asn Gly Asp Asp Gly Glu Ala Gly Lys Pro Gly Arg Pro Gly
       50                  55                  60
   Glu Arg Gly Pro Pro Gly Pro Gln Gly Ala Arg Gly Leu Pro Gly Thr
   65                  70                  75                  80
   Ala Gly Leu Pro Gly Met Lys Gly His Arg Gly Phe Ser Gly Leu Asp
                   85                  90                  95
   Gly Ala Lys Gly Asp Ala Gly Pro Ala Gly Pro Lys Gly Glu Pro Gly
               100                 105                 110
   Ser Pro Gly Glu Asn Gly Ala Pro Gly Gln Met Gly Pro Arg Gly Leu
           115                 120                 125
   Pro Gly Glu Arg Gly Arg Pro Gly Ala Pro Gly Pro Ala Gly Ala Arg
       130                 135                 140
   Gly Asn Asp Gly Ala Thr Gly Ala Ala Gly Pro Pro Gly Pro Thr Gly
   145                 150                 155                 160
   Pro Ala Gly Pro Pro Gly Phe Pro Gly Ala Val Gly Ala Lys Gly Glu
                   165                 170                 175
   Ala Gly Pro Gln Gly Pro Arg Gly Ser Glu Gly Pro Gln Gly Val Arg
               180                 185                 190
   Gly Glu Pro Gly Pro Pro Gly Pro Ala Gly Ala Ala Gly Pro Ala Gly
           195                 200                 205
   Asn Pro Gly Ala Asp Gly Gln Pro Gly Ala Lys Gly Ala Asn Gly Ala
       210                 215                 220
   Pro Gly Ile Ala Gly Ala Pro Gly Phe Pro Gly Ala Arg Gly Pro Ser
   225                 230                 235                 240
   Gly Pro Gln Gly Pro Gly Gly Pro Pro Gly Pro Lys Gly Asn Ser Gly
                   245                 250                 255
   Glu Pro Gly Ala Pro Gly Ser Lys Gly Asp Thr Gly Ala Lys Gly Glu
               260                 265                 270
   Pro Gly Pro Val Gly Val Gln Gly Pro Pro Gly Pro Ala Gly Glu Glu
           275                 280                 285
   Gly Lys Arg Gly Ala Arg Gly Glu Pro Gly Pro Thr Gly Leu Pro Gly
       290                 295                 300
   Pro Pro Gly Glu Arg Gly Gly Pro Gly Ser Arg Gly Phe Pro Gly Ala
   305                 310                 315                 320
   Asp Gly Val Ala Gly Pro Lys Gly Pro Ala Gly Glu Arg Gly Ser Pro
                   325                 330                 335
   Gly Pro Ala Gly Pro Lys Gly Ser Pro Gly Glu Ala Gly Arg Pro Gly
               340                 345                 350
   Glu Ala Gly Leu Pro Gly Ala Lys Gly Leu Thr Gly Ser Pro Gly Ser
           355                 360                 365
   Pro Gly Pro Asp Gly Lys Thr Gly Pro Pro Gly Pro Ala Gly Gln Asp
       370                 375                 380
   Gly Arg Pro Gly Pro Pro Gly Pro Pro Gly Ala Arg Gly Gln Ala Gly
   385                 390                 395                 400
   Val Met Gly Phe Pro Gly Pro Lys Gly Ala Ala Gly Glu Pro Gly Lys
                   405                 410                 415
   Ala Gly Glu Arg Gly Val Pro Gly Pro Pro Gly Ala Val Gly Pro Ala
   420 425 430
   Gly Lys Asp Gly Glu Ala Gly Ala Gln Gly Pro Pro Gly Pro Ala Gly
   435 440 445
   Pro Ala Gly Glu Arg Gly Glu Gln Gly Pro Ala Gly Ser Pro Gly Phe
   450 455 460
   Gln Gly Leu Pro Gly Pro Ala Gly Pro Pro Gly Glu Ala Gly Lys Pro
   465 470 475 480
   Gly Glu Gln Gly Val Pro Gly Asp Leu Gly Ala Pro Gly Pro Ser Gly
   485 490 495
   Ala Arg Gly Glu Arg Gly Phe Pro Gly Glu Arg Gly Val Gln Gly Pro
   500 505 510
   Pro Gly Pro Ala Gly Pro Arg Gly Ala Asn Gly Ala Pro Gly Asn Asp
   515 520 525
   Gly Ala Lys Gly Asp Ala Gly Ala Pro Gly Ala Pro Gly Ser Gln Gly
   530 535 540
   Ala Pro Gly Leu Gln Gly Met Pro Gly Glu Arg Gly Ala Ala Gly Leu
   545 550 555 560
   Pro Gly Pro Lys Gly Asp Arg Gly Asp Ala Gly Pro Lys Gly Ala Asp
   565 570 575
   Gly Ser Pro Gly Lys Asp Gly Val Arg Gly Leu Thr Gly Pro Ile Gly
   580 585 590
   Pro Pro Gly Pro Ala Gly Ala Pro Gly Asp Lys Gly Glu Ser Gly Pro
   595 600 605
   Ser Gly Pro Ala Gly Pro Thr Gly Ala Arg Gly Ala Pro Gly Asp Arg
   610 615 620
   Gly Glu Pro Gly Pro Pro Gly Pro Ala Gly Phe Ala Gly Pro Pro Gly
   625 630 635 640
   Ala Asp Gly Gln Pro Gly Ala Lys Gly Glu Pro Gly Asp Ala Gly Ala
   645 650 655
   Lys Gly Asp Ala Gly Pro Pro Gly Pro Ala Gly Pro Ala Gly Pro Pro
   660 665 670
   Gly Pro Ile Gly Asn Val Gly Ala Pro Gly Ala Lys Gly Ala Arg Gly
   675 680 685
   Ser Ala Gly Pro Pro Gly Ala Thr Gly Phe Pro Gly Ala Ala Gly Arg
       690                 695                 700
   Val Gly Pro Pro Gly Pro Ser Gly Asn Ala Gly Pro Pro Gly Pro Pro
   705                 710                 715                 720
   Gly Pro Ala Gly Lys Glu Gly Gly Lys Gly Pro Arg Gly Glu Thr Gly
                   725                 730                 735
   Pro Ala Gly Arg Pro Gly Glu Val Gly Pro Pro Gly Pro Pro Gly Pro
               740                 745                 750
   Ala Gly Glu Lys Gly Ser Pro Gly Ala Asp Gly Pro Ala Gly Ala Pro
           755                 760                 765
   Gly Thr Pro Gly Pro Gln Gly Ile Ala Gly Gln Arg Gly Val Val Gly
       770                 775                 780
   Leu Pro Gly Gln Arg Gly Glu Arg Gly Phe Pro Gly Leu Pro Gly Pro
   785                 790                 795                 800
   Ser Gly Glu Pro Gly Lys Gln Gly Pro Ser Gly Ala Ser Gly Glu Arg
                   805                 810                 815
   Gly Pro Pro Gly Pro Met Gly Pro Pro Gly Leu Ala Gly Pro Pro Gly
               820                 825                 830
   Glu Ser Gly Arg Glu Gly Ala Pro Gly Ala Glu Gly Ser Pro Gly Arg
           835                 840                 845
   Asp Gly Ser Pro Gly Ala Lys Gly Asp Arg Gly Glu Thr Gly Pro Ala
       850                 855                 860
   Gly Pro Pro Gly Ala Pro Gly Ala Pro Gly Ala Pro Gly Pro Val Gly
   865                 870                 875                 880
   Pro Ala Gly Lys Ser Gly Asp Arg Gly Glu Thr Gly Pro Ala Gly Pro
                   885                 890                 895
   Ala Gly Pro Val Gly Pro Ala Gly Ala Arg Gly Pro Ala Gly Pro Gln
               900                 905                 910
   Gly Pro Arg Gly Asp Lys Gly Glu Thr Gly Glu Gln Gly Asp Arg Gly
           915                 920                 925
   Ile Lys Gly His Arg Gly Phe Ser Gly Leu Gln Gly Pro Pro Gly Pro
       930                 935                 940
   Pro Gly Ser Pro Gly Glu Gln Gly Pro Ser Gly Ala Ser Gly Pro Ala
   945                 950                 955                 960
   Gly Pro Arg Gly Pro Pro Gly Ser Ala Gly Ala Pro Gly Lys Asp Gly
   965 970 975
   Leu Asn Gly Leu Pro Gly Pro Ile Gly Pro Pro Gly Pro Arg Gly Arg
   980 985 990
   Thr Gly Asp Ala Gly Pro Val Gly Pro Pro Gly Pro Pro Gly Pro Pro
   995 1000 1005
   Gly Pro Pro Gly Pro Pro
   1010
<210> 6
   <211> 1014
   <212> PRT
   <213> Homo sapiens
<400> 6
   Gly Pro Met Gly Pro Ser Gly Pro Arg Gly Leu Pro Gly Pro Pro Gly
   1               5                   10                  15
   Ala Pro Gly Pro Gln Gly Phe Gln Gly Pro Pro Gly Glu Pro Gly Glu
               20                  25                  30
   Pro Gly Ala Ser Gly Pro Met Gly Pro Pro Gly Pro Pro Gly Pro Pro
           35                  40                  45
   Gly Lys Asn Gly Asp Asp Gly Glu Ala Gly Lys Pro Gly Arg Pro Gly
       50                  55                  60
   Glu Arg Gly Pro Pro Gly Pro Gln Gly Ala Arg Gly Leu Pro Gly Thr
   65                  70                  75                  80
   Ala Gly Leu Pro Gly Met Lys Gly His Arg Gly Phe Ser Gly Leu Asp
                   85                  90                  95
   Gly Ala Lys Gly Asp Ala Gly Pro Ala Gly Pro Lys Gly Glu Pro Gly
               100                 105                 110
   Ser Pro Gly Glu Asn Gly Ala Pro Gly Gln Met Gly Pro Pro Gly Leu
           115                 120                 125
   Pro Gly Glu Arg Gly Arg Pro Gly Ala Pro Gly Pro Ala Gly Ala Arg
       130                 135                 140
   Gly Asn Asp Gly Ala Thr Gly Ala Ala Gly Pro Pro Gly Pro Thr Gly
   145                 150                 155                 160
   Pro Ala Gly Pro Pro Gly Phe Pro Gly Ala Val Gly Ala Lys Gly Glu
                   165                 170                 175
   Ala Gly Pro Gln Gly Pro Arg Gly Ser Glu Gly Pro Gln Gly Val Arg
   180 185 190
   Gly Glu Pro Gly Pro Pro Gly Pro Ala Gly Ala Ala Gly Pro Ala Gly
   195 200 205
   Asn Pro Gly Ala Asp Gly Gln Pro Gly Ala Lys Gly Ala Asn Gly Ala
   210 215 220
   Pro Gly Ile Ala Gly Ala Pro Gly Phe Pro Gly Ala Arg Gly Pro Ser
   225 230 235 240
   Gly Pro Gln Gly Pro Gly Gly Pro Pro Gly Pro Lys Gly Asn Ser Gly
   245 250 255
   Glu Pro Gly Ala Pro Gly Ser Lys Gly Asp Thr Gly Ala Lys Gly Glu
   260 265 270
   Pro Gly Pro Val Gly Val Gln Gly Pro Pro Gly Pro Ala Gly Glu Glu
   275 280 285
   Gly Lys Arg Gly Ala Arg Gly Glu Pro Gly Pro Thr Gly Leu Pro Gly
   290 295 300
   Pro Pro Gly Glu Arg Gly Gly Pro Gly Ser Arg Gly Phe Pro Gly Ala
   305 310 315 320
   Asp Gly Val Ala Gly Pro Lys Gly Pro Ala Gly Glu Arg Gly Ser Pro
   325 330 335
   Gly Pro Ala Gly Pro Lys Gly Ser Pro Gly Glu Ala Gly Arg Pro Gly
   340 345 350
   Glu Ala Gly Leu Pro Gly Ala Lys Gly Leu Thr Gly Ser Pro Gly Ser
   355 360 365
   Pro Gly Pro Asp Gly Lys Thr Gly Pro Pro Gly Pro Ala Gly Gln Asp
   370 375 380
   Gly Arg Pro Gly Pro Pro Gly Pro Pro Gly Ala Arg Gly Gln Ala Gly
   385 390 395 400
   Val Met Gly Phe Pro Gly Pro Lys Gly Ala Ala Gly Glu Pro Gly Lys
   405 410 415
   Ala Gly Glu Arg Gly Val Pro Gly Pro Pro Gly Ala Val Gly Pro Ala
   420 425 430
   Gly Lys Asp Gly Glu Ala Gly Ala Gln Gly Pro Pro Gly Pro Ala Gly
   435 440 445
   Pro Ala Gly Glu Arg Gly Glu Gln Gly Pro Ala Gly Ser Pro Gly Phe
   450 455 460
   Gln Gly Leu Pro Gly Pro Ala Gly Pro Pro Gly Glu Ala Gly Lys Pro
   465 470 475 480
   Gly Glu Gln Gly Val Pro Gly Asp Leu Gly Ala Pro Gly Pro Ser Gly
   485 490 495
   Ala Arg Gly Glu Arg Gly Phe Pro Gly Glu Arg Gly Val Gln Gly Pro
   500 505 510
   Pro Gly Pro Ala Gly Pro Arg Gly Ala Asn Gly Ala Pro Gly Asn Asp
   515 520 525
   Gly Ala Lys Gly Asp Ala Gly Ala Pro Gly Ala Pro Gly Ser Gln Gly
   530 535 540
   Ala Pro Gly Leu Gln Gly Met Pro Gly Glu Arg Gly Ala Ala Gly Leu
   545 550 555 560
   Pro Gly Pro Lys Gly Asp Arg Gly Asp Ala Gly Pro Lys Gly Ala Asp
   565 570 575
   Gly Ser Pro Gly Lys Asp Gly Val Arg Gly Leu Thr Gly Pro Ile Gly
   580 585 590
   Pro Pro Gly Pro Ala Gly Ala Pro Gly Asp Lys Gly Glu Ser Gly Pro
   595 600 605
   Ser Gly Pro Ala Gly Pro Thr Gly Ala Arg Gly Ala Pro Gly Asp Arg
   610 615 620
   Gly Glu Pro Gly Pro Pro Gly Pro Ala Gly Phe Ala Gly Pro Pro Gly
   625 630 635 640
   Ala Asp Gly Gln Pro Gly Ala Lys Gly Glu Pro Gly Asp Ala Gly Ala
   645 650 655
   Lys Gly Asp Ala Gly Pro Pro Gly Pro Ala Gly Pro Ala Gly Pro Pro
   660 665 670
   Gly Pro Ile Gly Asn Val Gly Ala Pro Gly Ala Lys Gly Ala Arg Gly
   675 680 685
   Ser Ala Gly Pro Pro Gly Ala Thr Gly Phe Pro Gly Ala Ala Gly Arg
   690 695 700
   Val Gly Pro Pro Gly Pro Ser Gly Asn Ala Gly Pro Pro Gly Pro Pro
   705 710 715 720
   Gly Pro Ala Gly Lys Glu Gly Gly Lys Gly Pro Arg Gly Glu Thr Gly
   725 730 735
   Pro Ala Gly Arg Pro Gly Glu Val Gly Pro Pro Gly Pro Pro Gly Pro
   740 745 750
   Ala Gly Glu Lys Gly Ser Pro Gly Ala Asp Gly Pro Ala Gly Ala Pro
   755 760 765
   Gly Thr Pro Gly Pro Gln Gly Ile Ala Gly Gln Arg Gly Val Val Gly
   770 775 780
   Leu Pro Gly Gln Arg Gly Glu Arg Gly Phe Pro Gly Leu Pro Gly Pro
   785 790 795 800
   Ser Gly Glu Pro Gly Lys Gln Gly Pro Ser Gly Ala Ser Gly Glu Arg
   805 810 815
   Gly Pro Pro Gly Pro Met Gly Pro Pro Gly Leu Ala Gly Pro Pro Gly
   820 825 830
   Glu Ser Gly Arg Glu Gly Ala Pro Gly Ala Glu Gly Ser Pro Gly Arg
   835 840 845
   Asp Gly Ser Pro Gly Ala Lys Gly Asp Arg Gly Glu Thr Gly Pro Ala
   850 855 860
   Gly Pro Pro Gly Ala Pro Gly Ala Pro Gly Ala Pro Gly Pro Val Gly
   865 870 875 880
   Pro Ala Gly Lys Ser Gly Asp Arg Gly Glu Thr Gly Pro Ala Gly Pro
   885 890 895
   Ala Gly Pro Val Gly Pro Ala Gly Ala Arg Gly Pro Ala Gly Pro Gln
   900 905 910
   Gly Pro Arg Gly Asp Lys Gly Glu Thr Gly Glu Gln Gly Asp Arg Gly
   915 920 925
   Ile Lys Gly His Arg Gly Phe Ser Gly Leu Gln Gly Pro Pro Gly Pro
   930 935 940
   Pro Gly Ser Pro Gly Glu Gln Gly Pro Ser Gly Ala Ser Gly Pro Ala
   945 950 955 960
   Gly Pro Arg Gly Pro Pro Gly Ser Ala Gly Ala Pro Gly Lys Asp Gly
   965 970 975
   Leu Asn Gly Leu Pro Gly Pro Ile Gly Pro Pro Gly Pro Arg Gly Arg
   980 985 990
   Thr Gly Asp Ala Gly Pro Val Gly Pro Pro Gly Pro Pro Gly Pro Pro
   995 1000 1005
   Gly Pro Pro Gly Pro Pro
   1010
<210> 7
   <211> 1014
   <212> PRT
   <213> Homo sapiens
<400> 7
   Gly Pro Met Gly Pro Ser Gly Pro Arg Gly Leu Pro Gly Pro Pro Gly
   1               5                   10                  15
   Ala Pro Gly Pro Gln Gly Phe Gln Gly Pro Pro Gly Glu Pro Gly Glu
               20                  25                  30
   Pro Gly Ala Ser Gly Pro Met Gly Pro Pro Gly Pro Pro Gly Pro Pro
           35                  40                  45
   Gly Lys Asn Gly Asp Asp Gly Glu Ala Gly Lys Pro Gly Arg Pro Gly
       50                  55                  60
   Glu Arg Gly Pro Pro Gly Pro Gln Gly Ala Arg Gly Leu Pro Gly Thr
   65                  70                  75                  80
   Ala Gly Leu Pro Gly Met Lys Gly His Arg Gly Phe Ser Gly Leu Asp
                   85                  90                  95
   Gly Ala Lys Gly Asp Ala Gly Pro Ala Gly Pro Lys Gly Glu Pro Gly
               100                 105                 110
   Ser Pro Gly Glu Asn Gly Ala Pro Gly Gln Met Gly Pro Pro Gly Leu
           115                 120                 125
   Pro Gly Glu Arg Gly Arg Pro Gly Ala Pro Gly Pro Ala Gly Ala Arg
       130                 135                 140
   Gly Asn Asp Gly Ala Thr Gly Ala Ala Gly Pro Pro Gly Pro Thr Gly
   145                 150                 155                 160
   Pro Ala Gly Pro Pro Gly Phe Pro Gly Ala Val Gly Ala Lys Gly Glu
                   165                 170                 175
   Ala Gly Pro Gln Gly Pro Arg Gly Ser Glu Gly Pro Gln Gly Val Arg
               180                 185                 190
   Gly Glu Pro Gly Pro Pro Gly Pro Ala Gly Ala Ala Gly Pro Ala Gly
           195                 200                 205
   Asn Pro Gly Ala Asp Gly Gln Pro Gly Ala Lys Gly Ala Asn Gly Ala
       210                 215                 220
   Pro Gly Ile Ala Gly Ala Pro Gly Phe Pro Gly Ala Arg Gly Pro Ser
   225                 230                 235                 240
   Gly Pro Gln Gly Pro Gly Gly Pro Pro Gly Pro Lys Gly Asn Ser Gly
                   245                 250                 255
   Glu Pro Gly Ala Pro Gly Ser Lys Gly Asp Thr Gly Ala Lys Gly Glu
               260                 265                 270
   Pro Gly Pro Val Gly Val Gln Gly Pro Pro Gly Pro Ala Gly Glu Glu
           275                 280                 285
   Gly Lys Arg Gly Ala Arg Gly Glu Pro Gly Pro Thr Gly Leu Pro Gly
       290                 295                 300
   Pro Pro Gly Glu Arg Gly Gly Pro Gly Ser Arg Gly Phe Pro Gly Ala
   305                 310                 315                 320
   Asp Gly Val Ala Gly Pro Lys Gly Pro Ala Gly Glu Arg Gly Ser Pro
                   325                 330                 335
   Gly Pro Ala Gly Pro Lys Gly Ser Pro Gly Glu Ala Gly Arg Pro Gly
               340                 345                 350
   Glu Ala Gly Leu Pro Gly Ala Lys Gly Leu Thr Gly Ser Pro Gly Ser
           355                 360                 365
   Pro Gly Pro Asp Gly Lys Thr Gly Pro Pro Gly Pro Ala Gly Gln Asp
       370                 375                 380
   Gly Arg Pro Gly Pro Pro Gly Pro Pro Gly Ala Arg Gly Gln Ala Gly
   385                 390                 395                 400
   Pro Pro Gly Phe Pro Gly Pro Lys Gly Ala Ala Gly Glu Pro Gly Lys
                   405                 410                 415
   Ala Gly Glu Arg Gly Val Pro Gly Pro Pro Gly Ala Val Gly Pro Ala
               420                 425                 430
   Gly Lys Asp Gly Glu Ala Gly Ala Gln Gly Pro Pro Gly Pro Ala Gly
           435                 440                 445
   Pro Ala Gly Glu Arg Gly Glu Gln Gly Pro Ala Gly Ser Pro Gly Phe
       450                 455                 460
   Gln Gly Leu Pro Gly Pro Ala Gly Pro Pro Gly Glu Ala Gly Lys Pro
   465                 470                 475                 480
   Gly Glu Gln Gly Val Pro Gly Asp Leu Gly Ala Pro Gly Pro Ser Gly
   485 490 495
   Ala Arg Gly Glu Arg Gly Phe Pro Gly Glu Arg Gly Val Gln Gly Pro
   500 505 510
   Pro Gly Pro Ala Gly Pro Arg Gly Ala Asn Gly Ala Pro Gly Asn Asp
   515 520 525
   Gly Ala Lys Gly Asp Ala Gly Ala Pro Gly Ala Pro Gly Ser Gln Gly
   530 535 540
   Ala Pro Gly Leu Gln Gly Met Pro Gly Glu Arg Gly Ala Ala Gly Leu
   545 550 555 560
   Pro Gly Pro Lys Gly Asp Arg Gly Asp Ala Gly Pro Lys Gly Ala Asp
   565 570 575
   Gly Ser Pro Gly Lys Asp Gly Val Arg Gly Leu Thr Gly Pro Ile Gly
   580 585 590
   Pro Pro Gly Pro Ala Gly Ala Pro Gly Asp Lys Gly Glu Ser Gly Pro
   595 600 605
   Ser Gly Pro Ala Gly Pro Thr Gly Ala Arg Gly Ala Pro Gly Asp Arg
   610 615 620
   Gly Glu Pro Gly Pro Pro Gly Pro Ala Gly Phe Ala Gly Pro Pro Gly
   625 630 635 640
   Ala Asp Gly Gln Pro Gly Ala Lys Gly Glu Pro Gly Asp Ala Gly Ala
   645 650 655
   Lys Gly Asp Ala Gly Pro Pro Gly Pro Ala Gly Pro Ala Gly Pro Pro
   660 665 670
   Gly Pro Ile Gly Asn Val Gly Ala Pro Gly Ala Lys Gly Ala Arg Gly
   675 680 685
   Ser Ala Gly Pro Pro Gly Ala Thr Gly Phe Pro Gly Ala Ala Gly Arg
   690 695 700
   Val Gly Pro Pro Gly Pro Ser Gly Asn Ala Gly Pro Pro Gly Pro Pro
   705 710 715 720
   Gly Pro Ala Gly Lys Glu Gly Gly Lys Gly Pro Arg Gly Glu Thr Gly
   725 730 735
   Pro Ala Gly Arg Pro Gly Glu Val Gly Pro Pro Gly Pro Pro Gly Pro
   740 745 750
   Ala Gly Glu Lys Gly Ser Pro Gly Ala Asp Gly Pro Ala Gly Ala Pro
   755 760 765
   Gly Thr Pro Gly Pro Gln Gly Ile Ala Gly Gln Arg Gly Val Val Gly
   770 775 780
   Leu Pro Gly Gln Arg Gly Glu Arg Gly Phe Pro Gly Leu Pro Gly Pro
   785 790 795 800
   Ser Gly Glu Pro Gly Lys Gln Gly Pro Ser Gly Ala Ser Gly Glu Arg
   805 810 815
   Gly Pro Pro Gly Pro Met Gly Pro Pro Gly Leu Ala Gly Pro Pro Gly
   820 825 830
   Glu Ser Gly Arg Glu Gly Ala Pro Gly Ala Glu Gly Ser Pro Gly Arg
   835 840 845
   Asp Gly Ser Pro Gly Ala Lys Gly Asp Arg Gly Glu Thr Gly Pro Ala
   850 855 860
   Gly Pro Pro Gly Ala Pro Gly Ala Pro Gly Ala Pro Gly Pro Val Gly
   865 870 875 880
   Pro Ala Gly Lys Ser Gly Asp Arg Gly Glu Thr Gly Pro Ala Gly Pro
   885 890 895
   Ala Gly Pro Val Gly Pro Ala Gly Ala Arg Gly Pro Ala Gly Pro Gln
   900 905 910
   Gly Pro Arg Gly Asp Lys Gly Glu Thr Gly Glu Gln Gly Asp Arg Gly
   915 920 925
   Ile Lys Gly His Arg Gly Phe Ser Gly Leu Gln Gly Pro Pro Gly Pro
   930 935 940
   Pro Gly Ser Pro Gly Glu Gln Gly Pro Ser Gly Ala Ser Gly Pro Ala
   945 950 955 960
   Gly Pro Arg Gly Pro Pro Gly Ser Ala Gly Ala Pro Gly Lys Asp Gly
   965 970 975
   Leu Asn Gly Leu Pro Gly Pro Ile Gly Pro Pro Gly Pro Arg Gly Arg
   980 985 990
   Thr Gly Asp Ala Gly Pro Val Gly Pro Pro Gly Pro Pro Gly Pro Pro
   995 1000 1005
   Gly Pro Pro Gly Pro Pro
   1010
<210> 8
   <211> 1014
   <212> PRT
   <213> Homo sapiens
<400> 8
   Gly Pro Met Gly Pro Ser Gly Pro Arg Gly Leu Pro Gly Pro Pro Gly
   1               5                   10                  15
   Ala Pro Gly Pro Gln Gly Phe Gln Gly Pro Pro Gly Glu Pro Gly Glu
               20                  25                  30
   Pro Gly Ala Ser Gly Pro Met Gly Pro Pro Gly Pro Pro Gly Pro Pro
           35                  40                  45
   Gly Lys Asn Gly Asp Asp Gly Glu Ala Gly Lys Pro Gly Arg Pro Gly
       50                  55                  60
   Glu Arg Gly Pro Pro Gly Pro Gln Gly Ala Arg Gly Leu Pro Gly Thr
   65                  70                  75                  80
   Ala Gly Leu Pro Gly Met Lys Gly His Arg Gly Phe Ser Gly Leu Asp
                   85                  90                  95
   Gly Ala Lys Gly Asp Ala Gly Pro Ala Gly Pro Lys Gly Glu Pro Gly
               100                 105                 110
   Ser Pro Gly Glu Asn Gly Ala Pro Gly Gln Met Gly Pro Pro Gly Leu
           115                 120                 125
   Pro Gly Glu Arg Gly Arg Pro Gly Ala Pro Gly Pro Ala Gly Ala Arg
       130                 135                 140
   Gly Asn Asp Gly Ala Thr Gly Ala Ala Gly Pro Pro Gly Pro Thr Gly
   145                 150                 155                 160
   Pro Ala Gly Pro Pro Gly Phe Pro Gly Ala Val Gly Ala Lys Gly Glu
                   165                 170                 175
   Ala Gly Pro Gln Gly Pro Arg Gly Ser Glu Gly Pro Gln Gly Val Arg
               180                 185                 190
   Gly Glu Pro Gly Pro Pro Gly Pro Ala Gly Ala Ala Gly Pro Ala Gly
           195                 200                 205
   Asn Pro Gly Ala Asp Gly Gln Pro Gly Ala Lys Gly Ala Asn Gly Ala
       210                 215                 220
   Pro Gly Ile Ala Gly Ala Pro Gly Phe Pro Gly Ala Arg Gly Pro Ser
   225                 230                 235                 240
   Gly Pro Gln Gly Pro Gly Gly Pro Pro Gly Pro Lys Gly Asn Ser Gly
   245 250 255
   Glu Pro Gly Ala Pro Gly Ser Lys Gly Asp Thr Gly Ala Lys Gly Glu
   260 265 270
   Pro Gly Pro Val Gly Val Gln Gly Pro Pro Gly Pro Ala Gly Glu Glu
   275 280 285
   Gly Lys Arg Gly Ala Arg Gly Glu Pro Gly Pro Thr Gly Leu Pro Gly
   290 295 300
   Pro Pro Gly Glu Arg Gly Gly Pro Gly Ser Arg Gly Phe Pro Gly Ala
   305 310 315 320
   Asp Gly Val Ala Gly Pro Lys Gly Pro Ala Gly Glu Arg Gly Ser Pro
   325 330 335
   Gly Pro Ala Gly Pro Lys Gly Ser Pro Gly Glu Ala Gly Arg Pro Gly
   340 345 350
   Glu Ala Gly Leu Pro Gly Ala Lys Gly Leu Thr Gly Ser Pro Gly Ser
   355 360 365
   Pro Gly Pro Asp Gly Lys Thr Gly Pro Pro Gly Pro Ala Gly Gln Asp
   370 375 380
   Gly Arg Pro Gly Pro Pro Gly Pro Pro Gly Ala Arg Gly Gln Ala Gly
   385 390 395 400
   Pro Pro Gly Phe Pro Gly Pro Lys Gly Ala Ala Gly Glu Pro Gly Lys
   405 410 415
   Ala Gly Glu Arg Gly Val Pro Gly Pro Pro Gly Ala Val Gly Pro Ala
   420 425 430
   Gly Lys Asp Gly Glu Ala Gly Ala Gln Gly Pro Pro Gly Pro Ala Gly
   435 440 445
   Pro Ala Gly Glu Arg Gly Glu Gln Gly Pro Ala Gly Ser Pro Gly Phe
   450 455 460
   Gln Gly Leu Pro Gly Pro Ala Gly Pro Pro Gly Glu Ala Gly Lys Pro
   465 470 475 480
   Gly Glu Gln Gly Val Pro Gly Asp Leu Gly Ala Pro Gly Pro Ser Gly
   485 490 495
   Ala Arg Gly Glu Arg Gly Phe Pro Gly Glu Arg Gly Val Gln Gly Pro
   500 505 510
   Pro Gly Pro Ala Gly Pro Arg Gly Ala Asn Gly Ala Pro Gly Asn Asp
   515 520 525
   Gly Ala Lys Gly Asp Ala Gly Ala Pro Gly Ala Pro Gly Ser Gln Gly
   530 535 540
   Ala Pro Gly Leu Gln Gly Met Pro Gly Glu Arg Gly Ala Ala Gly Leu
   545 550 555 560
   Pro Gly Pro Lys Gly Asp Arg Gly Asp Ala Gly Pro Lys Gly Ala Asp
   565 570 575
   Gly Ser Pro Gly Lys Asp Gly Val Arg Gly Leu Thr Gly Pro Ile Gly
   580 585 590
   Pro Pro Gly Pro Ala Gly Ala Pro Gly Pro Pro Gly Glu Ser Gly Pro
   595 600 605
   Ser Gly Pro Ala Gly Pro Thr Gly Ala Arg Gly Ala Pro Gly Asp Arg
   610 615 620
   Gly Glu Pro Gly Pro Pro Gly Pro Ala Gly Phe Ala Gly Pro Pro Gly
   625 630 635 640
   Ala Asp Gly Gln Pro Gly Ala Lys Gly Glu Pro Gly Asp Ala Gly Ala
   645 650 655
   Lys Gly Asp Ala Gly Pro Pro Gly Pro Ala Gly Pro Ala Gly Pro Pro
   660 665 670
   Gly Pro Ile Gly Pro Pro Gly Ala Pro Gly Ala Lys Gly Ala Arg Gly
   675 680 685
   Ser Ala Gly Pro Pro Gly Ala Thr Gly Phe Pro Gly Ala Ala Gly Arg
   690 695 700
   Val Gly Pro Pro Gly Pro Ser Gly Asn Ala Gly Pro Pro Gly Pro Pro
   705 710 715 720
   Gly Pro Ala Gly Lys Glu Gly Gly Lys Gly Pro Arg Gly Glu Thr Gly
   725 730 735
   Pro Ala Gly Arg Pro Gly Glu Val Gly Pro Pro Gly Pro Pro Gly Pro
   740 745 750
   Ala Gly Glu Lys Gly Ser Pro Gly Ala Asp Gly Pro Ala Gly Ala Pro
   755 760 765
   Gly Thr Pro Gly Pro Gln Gly Ile Ala Gly Gln Arg Gly Val Val Gly
   770 775 780
   Leu Pro Gly Gln Arg Gly Glu Arg Gly Phe Pro Gly Leu Pro Gly Pro
   785 790 795 800
   Ser Gly Glu Pro Gly Lys Gln Gly Pro Ser Gly Ala Ser Gly Glu Arg
   805 810 815
   Gly Pro Pro Gly Pro Met Gly Pro Pro Gly Leu Ala Gly Pro Pro Gly
   820 825 830
   Glu Ser Gly Arg Glu Gly Ala Pro Gly Ala Glu Gly Ser Pro Gly Arg
   835 840 845
   Asp Gly Ser Pro Gly Ala Lys Gly Asp Arg Gly Glu Thr Gly Pro Ala
   850 855 860
   Gly Pro Pro Gly Ala Pro Gly Ala Pro Gly Ala Pro Gly Pro Val Gly
   865 870 875 880
   Pro Ala Gly Lys Ser Gly Asp Arg Gly Glu Thr Gly Pro Ala Gly Pro
   885 890 895
   Ala Gly Pro Val Gly Pro Ala Gly Ala Arg Gly Pro Ala Gly Pro Gln
   900 905 910
   Gly Pro Arg Gly Asp Lys Gly Glu Thr Gly Glu Gln Gly Asp Arg Gly
   915 920 925
   Ile Lys Gly His Arg Gly Phe Ser Gly Leu Gln Gly Pro Pro Gly Pro
   930 935 940
   Pro Gly Ser Pro Gly Glu Gln Gly Pro Ser Gly Ala Ser Gly Pro Ala
   945 950 955 960
   Gly Pro Arg Gly Pro Pro Gly Ser Ala Gly Ala Pro Gly Lys Asp Gly
   965 970 975
   Leu Asn Gly Leu Pro Gly Pro Ile Gly Pro Pro Gly Pro Arg Gly Arg
   980 985 990
   Thr Gly Asp Ala Gly Pro Val Gly Pro Pro Gly Pro Pro Gly Pro Pro
   995 1000 1005
   Gly Pro Pro Gly Pro Pro
   1010
<210> 9
   <211> 1014
   <212> PRT
   <213> Homo sapiens
<400> 9
   Gly Pro Met Gly Pro Ser Gly Pro Arg Gly Leu Pro Gly Pro Pro Gly
   1               5                   10                  15
   Ala Pro Gly Pro Gln Gly Phe Gln Gly Pro Pro Gly Glu Pro Gly Glu
   20 25 30
   Pro Gly Ala Ser Gly Pro Met Gly Pro Pro Gly Pro Pro Gly Pro Pro
   35 40 45
   Gly Lys Asn Gly Asp Asp Gly Glu Ala Gly Lys Pro Gly Arg Pro Gly
   50 55 60
   Glu Arg Gly Pro Pro Gly Pro Gln Gly Ala Arg Gly Leu Pro Gly Thr
   65 70 75 80
   Ala Gly Leu Pro Gly Met Lys Gly His Arg Gly Phe Ser Gly Leu Asp
   85 90 95
   Gly Ala Lys Gly Asp Ala Gly Pro Ala Gly Pro Lys Gly Glu Pro Gly
   100 105 110
   Ser Pro Gly Glu Asn Gly Ala Pro Gly Gln Met Gly Pro Pro Gly Leu
   115 120 125
   Pro Gly Glu Arg Gly Arg Pro Gly Ala Pro Gly Pro Ala Gly Ala Arg
   130 135 140
   Gly Asn Asp Gly Ala Thr Gly Ala Ala Gly Pro Pro Gly Pro Thr Gly
   145 150 155 160
   Pro Ala Gly Pro Pro Gly Phe Pro Gly Ala Val Gly Ala Lys Gly Glu
   165 170 175
   Ala Gly Pro Gln Gly Pro Arg Gly Ser Glu Gly Pro Gln Gly Val Arg
   180 185 190
   Gly Glu Pro Gly Pro Pro Gly Pro Ala Gly Ala Ala Gly Pro Ala Gly
   195 200 205
   Asn Pro Gly Ala Asp Gly Gln Pro Gly Ala Lys Gly Ala Asn Gly Ala
   210 215 220
   Pro Gly Ile Ala Gly Ala Pro Gly Phe Pro Gly Ala Arg Gly Pro Ser
   225 230 235 240
   Gly Pro Gln Gly Pro Gly Gly Pro Pro Gly Pro Lys Gly Asn Ser Gly
   245 250 255
   Glu Pro Gly Ala Pro Gly Ser Lys Gly Asp Thr Gly Ala Lys Gly Glu
   260 265 270
   Pro Gly Pro Val Gly Val Gln Gly Pro Pro Gly Pro Ala Gly Glu Glu
   275 280 285
   Gly Lys Arg Gly Ala Arg Gly Glu Pro Gly Pro Thr Gly Leu Pro Gly
       290                 295                 300
   Pro Pro Gly Glu Arg Gly Gly Pro Gly Ser Arg Gly Phe Pro Gly Ala
   305                 310                 315                 320
   Asp Gly Val Ala Gly Pro Lys Gly Pro Ala Gly Glu Arg Gly Ser Pro
                   325                 330                 335
   Gly Pro Ala Gly Pro Lys Gly Ser Pro Gly Glu Ala Gly Arg Pro Gly
               340                 345                 350
   Glu Ala Gly Leu Pro Gly Ala Lys Gly Leu Thr Gly Ser Pro Gly Ser
           355                 360                 365
   Pro Gly Pro Asp Gly Lys Thr Gly Pro Pro Gly Pro Ala Gly Gln Asp
       370                 375                 380
   Gly Arg Pro Gly Pro Pro Gly Pro Pro Gly Ala Arg Gly Gln Ala Gly
   385                 390                 395                 400
   Val Met Gly Phe Pro Gly Pro Lys Gly Ala Ala Gly Glu Pro Gly Lys
                   405                 410                 415
   Ala Gly Glu Arg Gly Val Pro Gly Pro Pro Gly Ala Val Gly Pro Ala
               420                 425                 430
   Gly Lys Asp Gly Glu Ala Gly Ala Gln Gly Pro Pro Gly Pro Ala Gly
           435                 440                 445
   Pro Ala Gly Glu Arg Gly Glu Gln Gly Pro Ala Gly Ser Pro Gly Phe
       450                 455                 460
   Gln Gly Leu Pro Gly Pro Ala Gly Pro Pro Gly Glu Ala Gly Lys Pro
   465                 470                 475                 480
   Gly Glu Gln Gly Val Pro Gly Asp Leu Gly Ala Pro Gly Pro Ser Gly
                   485                 490                 495
   Ala Arg Gly Glu Arg Gly Phe Pro Gly Glu Arg Gly Val Gln Gly Pro
               500                 505                 510
   Pro Gly Pro Ala Gly Pro Arg Gly Ala Asn Gly Ala Pro Gly Asn Asp
           515                 520                 525
   Gly Ala Lys Gly Asp Ala Gly Ala Pro Gly Ala Pro Gly Ser Gln Gly
       530                 535                 540
   Ala Pro Gly Leu Gln Gly Met Pro Gly Glu Arg Gly Ala Ala Gly Leu
   545                 550                 555                 560
   Pro Gly Pro Lys Gly Asp Arg Gly Asp Ala Gly Pro Lys Gly Ala Asp
   565 570 575
   Gly Ser Pro Gly Lys Asp Gly Val Arg Gly Leu Thr Gly Pro Ile Gly
   580 585 590
   Pro Pro Gly Pro Ala Gly Ala Pro Gly Pro Pro Gly Glu Ser Gly Pro
   595 600 605
   Ser Gly Pro Ala Gly Pro Thr Gly Ala Arg Gly Ala Pro Gly Asp Arg
   610 615 620
   Gly Glu Pro Gly Pro Pro Gly Pro Ala Gly Phe Ala Gly Pro Pro Gly
   625 630 635 640
   Ala Asp Gly Gln Pro Gly Ala Lys Gly Glu Pro Gly Asp Ala Gly Ala
   645 650 655
   Lys Gly Asp Ala Gly Pro Pro Gly Pro Ala Gly Pro Ala Gly Pro Pro
   660 665 670
   Gly Pro Ile Gly Pro Pro Gly Ala Pro Gly Ala Lys Gly Ala Arg Gly
   675 680 685
   Ser Ala Gly Pro Pro Gly Ala Thr Gly Phe Pro Gly Ala Ala Gly Arg
   690 695 700
   Val Gly Pro Pro Gly Pro Ser Gly Asn Ala Gly Pro Pro Gly Pro Pro
   705 710 715 720
   Gly Pro Ala Gly Lys Glu Gly Gly Lys Gly Pro Arg Gly Glu Thr Gly
   725 730 735
   Pro Ala Gly Arg Pro Gly Glu Val Gly Pro Pro Gly Pro Pro Gly Pro
   740 745 750
   Ala Gly Glu Lys Gly Ser Pro Gly Ala Asp Gly Pro Ala Gly Ala Pro
   755 760 765
   Gly Thr Pro Gly Pro Gln Gly Ile Ala Gly Gln Arg Gly Val Val Gly
   770 775 780
   Leu Pro Gly Gln Arg Gly Glu Arg Gly Phe Pro Gly Leu Pro Gly Pro
   785 790 795 800
   Ser Gly Glu Pro Gly Lys Gln Gly Pro Ser Gly Ala Ser Gly Glu Arg
   805 810 815
   Gly Pro Pro Gly Pro Met Gly Pro Pro Gly Leu Ala Gly Pro Pro Gly
   820 825 830
   Glu Ser Gly Arg Glu Gly Ala Pro Gly Ala Glu Gly Ser Pro Gly Arg
   835 840 845
   Asp Gly Ser Pro Gly Ala Lys Gly Asp Arg Gly Glu Thr Gly Pro Ala
   850 855 860
   Gly Pro Pro Gly Ala Pro Gly Ala Pro Gly Ala Pro Gly Pro Val Gly
   865 870 875 880
   Pro Ala Gly Lys Ser Gly Asp Arg Gly Glu Thr Gly Pro Ala Gly Pro
   885 890 895
   Ala Gly Pro Val Gly Pro Ala Gly Ala Arg Gly Pro Ala Gly Pro Gln
   900 905 910
   Gly Pro Arg Gly Asp Lys Gly Glu Thr Gly Glu Gln Gly Asp Arg Gly
   915 920 925
   Ile Lys Gly His Arg Gly Phe Ser Gly Leu Gln Gly Pro Pro Gly Pro
   930 935 940
   Pro Gly Ser Pro Gly Glu Gln Gly Pro Ser Gly Ala Ser Gly Pro Ala
   945 950 955 960
   Gly Pro Arg Gly Pro Pro Gly Ser Ala Gly Ala Pro Gly Lys Asp Gly
   965 970 975
   Leu Asn Gly Leu Pro Gly Pro Ile Gly Pro Pro Gly Pro Arg Gly Arg
   980 985 990
   Thr Gly Asp Ala Gly Pro Val Gly Pro Pro Gly Pro Pro Gly Pro Pro
   995 1000 1005
   Gly Pro Pro Gly Pro Pro
   1010
<210> 10
   <211> 1014
   <212> PRT
   <213> Homo sapiens
<400> 10
   Gly Pro Met Gly Pro Ser Gly Pro Arg Gly Leu Pro Gly Pro Pro Gly
   1               5                   10                  15
   Ala Pro Gly Pro Gln Gly Phe Gln Gly Pro Pro Gly Glu Pro Gly Glu
               20                  25                  30
   Pro Gly Ala Ser Gly Pro Met Gly Pro Pro Gly Pro Pro Gly Pro Pro
           35                  40                  45
   Gly Lys Asn Gly Asp Asp Gly Glu Ala Gly Lys Pro Gly Arg Pro Gly
       50                  55                  60
   Glu Arg Gly Pro Pro Gly Pro Gln Gly Ala Arg Gly Leu Pro Gly Thr
   65                  70                  75                  80
   Ala Gly Leu Pro Gly Met Lys Gly His Arg Gly Phe Ser Gly Leu Asp
                   85                  90                  95
   Gly Ala Lys Gly Asp Ala Gly Pro Ala Gly Pro Lys Gly Glu Pro Gly
               100                 105                 110
   Ser Pro Gly Glu Asn Gly Ala Pro Gly Gln Met Gly Pro Pro Gly Leu
           115                 120                 125
   Pro Gly Glu Arg Gly Arg Pro Gly Ala Pro Gly Pro Ala Gly Ala Arg
       130                 135                 140
   Gly Asn Asp Gly Ala Thr Gly Ala Ala Gly Pro Pro Gly Pro Thr Gly
   145                 150                 155                 160
   Pro Ala Gly Pro Pro Gly Phe Pro Gly Ala Val Gly Ala Lys Gly Glu
                   165                 170                 175
   Ala Gly Pro Gln Gly Pro Arg Gly Ser Glu Gly Pro Gln Gly Val Arg
               180                 185                 190
   Gly Glu Pro Gly Pro Pro Gly Pro Ala Gly Ala Ala Gly Pro Ala Gly
           195                 200                 205
   Asn Pro Gly Ala Asp Gly Gln Pro Gly Ala Lys Gly Ala Asn Gly Ala
       210                 215                 220
   Pro Gly Ile Ala Gly Ala Pro Gly Phe Pro Gly Ala Arg Gly Pro Ser
   225                 230                 235                 240
   Gly Pro Gln Gly Pro Gly Gly Pro Pro Gly Pro Lys Gly Asn Ser Gly
                   245                 250                 255
   Glu Pro Gly Ala Pro Gly Ser Lys Gly Asp Thr Gly Ala Lys Gly Glu
               260                 265                 270
   Pro Gly Pro Val Gly Val Gln Gly Pro Pro Gly Pro Ala Gly Glu Glu
           275                 280                 285
   Gly Lys Arg Gly Ala Arg Gly Glu Pro Gly Pro Thr Gly Leu Pro Gly
       290                 295                 300
   Pro Pro Gly Glu Arg Gly Gly Pro Gly Ser Arg Gly Phe Pro Gly Ala
   305                 310                 315                 320
   Asp Gly Val Ala Gly Pro Lys Gly Pro Ala Gly Glu Arg Gly Ser Pro
   325 330 335
   Gly Pro Ala Gly Pro Lys Gly Ser Pro Gly Glu Ala Gly Arg Pro Gly
   340 345 350
   Glu Ala Gly Leu Pro Gly Ala Lys Gly Leu Thr Gly Ser Pro Gly Ser
   355 360 365
   Pro Gly Pro Asp Gly Lys Thr Gly Pro Pro Gly Pro Ala Gly Gln Asp
   370 375 380
   Gly Arg Pro Gly Pro Pro Gly Pro Pro Gly Ala Arg Gly Gln Ala Gly
   385 390 395 400
   Val Met Gly Phe Pro Gly Pro Lys Gly Ala Ala Gly Glu Pro Gly Lys
   405 410 415
   Ala Gly Glu Arg Gly Val Pro Gly Pro Pro Gly Ala Val Gly Pro Ala
   420 425 430
   Gly Lys Asp Gly Glu Ala Gly Ala Gln Gly Pro Pro Gly Pro Ala Gly
   435 440 445
   Pro Ala Gly Glu Arg Gly Glu Gln Gly Pro Ala Gly Ser Pro Gly Phe
   450 455 460
   Gln Gly Leu Pro Gly Pro Ala Gly Pro Pro Gly Glu Ala Gly Lys Pro
   465 470 475 480
   Gly Glu Gln Gly Val Pro Gly Asp Leu Gly Ala Pro Gly Pro Ser Gly
   485 490 495
   Ala Arg Gly Glu Arg Gly Phe Pro Gly Glu Arg Gly Val Gln Gly Pro
   500 505 510
   Pro Gly Pro Ala Gly Pro Arg Gly Ala Asn Gly Ala Pro Gly Asn Asp
   515 520 525
   Gly Ala Lys Gly Asp Ala Gly Ala Pro Gly Ala Pro Gly Ser Gln Gly
   530 535 540
   Ala Pro Gly Leu Gln Gly Met Pro Gly Glu Arg Gly Ala Ala Gly Leu
   545 550 555 560
   Pro Gly Pro Lys Gly Asp Arg Gly Asp Ala Gly Pro Lys Gly Ala Asp
   565 570 575
   Gly Ser Pro Gly Lys Asp Gly Val Arg Gly Leu Thr Gly Pro Ile Gly
   580 585 590
   Pro Pro Gly Pro Ala Gly Ala Pro Gly Asp Lys Gly Glu Ser Gly Pro
   595 600 605
   Ser Gly Pro Ala Gly Pro Thr Gly Ala Arg Gly Ala Pro Gly Asp Arg
   610 615 620
   Gly Glu Pro Gly Pro Pro Gly Pro Ala Gly Phe Ala Gly Pro Pro Gly
   625 630 635 640
   Ala Asp Gly Gln Pro Gly Ala Lys Gly Glu Pro Gly Asp Ala Gly Ala
   645 650 655
   Lys Gly Asp Ala Gly Pro Pro Gly Pro Ala Gly Pro Ala Gly Pro Pro
   660 665 670
   Gly Pro Ile Gly Pro Pro Gly Ala Pro Gly Ala Lys Gly Ala Arg Gly
   675 680 685
   Ser Ala Gly Pro Pro Gly Ala Thr Gly Phe Pro Gly Ala Ala Gly Arg
   690 695 700
   Val Gly Pro Pro Gly Pro Ser Gly Asn Ala Gly Pro Pro Gly Pro Pro
   705 710 715 720
   Gly Pro Ala Gly Lys Glu Gly Gly Lys Gly Pro Arg Gly Glu Thr Gly
   725 730 735
   Pro Ala Gly Arg Pro Gly Glu Val Gly Pro Pro Gly Pro Pro Gly Pro
   740 745 750
   Ala Gly Glu Lys Gly Ser Pro Gly Ala Asp Gly Pro Ala Gly Ala Pro
   755 760 765
   Gly Thr Pro Gly Pro Gln Gly Ile Ala Gly Gln Arg Gly Val Val Gly
   770 775 780
   Leu Pro Gly Gln Arg Gly Glu Arg Gly Phe Pro Gly Leu Pro Gly Pro
   785 790 795 800
   Ser Gly Glu Pro Gly Lys Gln Gly Pro Ser Gly Ala Ser Gly Glu Arg
   805 810 815
   Gly Pro Pro Gly Pro Met Gly Pro Pro Gly Leu Ala Gly Pro Pro Gly
   820 825 830
   Glu Ser Gly Arg Glu Gly Ala Pro Gly Ala Glu Gly Ser Pro Gly Arg
   835 840 845
   Asp Gly Ser Pro Gly Ala Lys Gly Asp Arg Gly Glu Thr Gly Pro Ala
   850 855 860
   Gly Pro Pro Gly Ala Pro Gly Ala Pro Gly Ala Pro Gly Pro Val Gly
   865 870 875 880
   Pro Ala Gly Lys Ser Gly Asp Arg Gly Glu Thr Gly Pro Ala Gly Pro
   885 890 895
   Ala Gly Pro Val Gly Pro Ala Gly Ala Arg Gly Pro Ala Gly Pro Gln
   900 905 910
   Gly Pro Arg Gly Asp Lys Gly Glu Thr Gly Glu Gln Gly Asp Arg Gly
   915 920 925
   Ile Lys Gly His Arg Gly Phe Ser Gly Leu Gln Gly Pro Pro Gly Pro
   930 935 940
   Pro Gly Ser Pro Gly Glu Gln Gly Pro Ser Gly Ala Ser Gly Pro Ala
   945 950 955 960
   Gly Pro Arg Gly Pro Pro Gly Ser Ala Gly Ala Pro Gly Lys Asp Gly
   965 970 975
   Leu Asn Gly Leu Pro Gly Pro Ile Gly Pro Pro Gly Pro Arg Gly Arg
   980 985 990
   Thr Gly Asp Ala Gly Pro Val Gly Pro Pro Gly Pro Pro Gly Pro Pro
   995 1000 1005
   Gly Pro Pro Gly Pro Pro
   1010
<210> 11
   <211> 1014
   <212> PRT
   <213> Homo sapiens
<400> 11
   Gly Pro Met Gly Pro Ser Gly Pro Arg Gly Leu Pro Gly Pro Pro Gly
   1 5 10 15
   Ala Pro Gly Pro Gln Gly Phe Gln Gly Pro Pro Gly Glu Pro Gly Glu
   20 25 30
   Pro Gly Ala Ser Gly Pro Met Gly Pro Pro Gly Pro Pro Gly Pro Pro
   35 40 45
   Gly Lys Asn Gly Asp Asp Gly Glu Ala Gly Lys Pro Gly Arg Pro Gly
   50 55 60
   Glu Arg Gly Pro Pro Gly Pro Gln Gly Ala Arg Gly Leu Pro Gly Thr
   65 70 75 80
   Ala Gly Leu Pro Gly Met Lys Gly His Arg Gly Phe Ser Gly Leu Asp
                   85                  90                  95
   Gly Ala Lys Gly Asp Ala Gly Pro Ala Gly Pro Lys Gly Glu Pro Gly
               100                 105                 110
   Ser Pro Gly Glu Asn Gly Ala Pro Gly Gln Met Gly Pro Pro Gly Leu
           115                 120                 125
   Pro Gly Glu Arg Gly Arg Pro Gly Ala Pro Gly Pro Ala Gly Ala Arg
       130                 135                 140
   Gly Asn Asp Gly Ala Thr Gly Ala Ala Gly Pro Pro Gly Pro Thr Gly
   145                 150                 155                 160
   Pro Ala Gly Pro Pro Gly Phe Pro Gly Ala Val Gly Ala Lys Gly Glu
                   165                 170                 175
   Ala Gly Pro Gln Gly Pro Arg Gly Ser Glu Gly Pro Gln Gly Val Arg
               180                 185                 190
   Gly Glu Pro Gly Pro Pro Gly Pro Ala Gly Ala Ala Gly Pro Ala Gly
           195                 200                 205
   Asn Pro Gly Ala Asp Gly Gln Pro Gly Ala Lys Gly Ala Asn Gly Ala
       210                 215                 220
   Pro Gly Ile Ala Gly Ala Pro Gly Phe Pro Gly Ala Arg Gly Pro Ser
   225                 230                 235                 240
   Gly Pro Gln Gly Pro Gly Gly Pro Pro Gly Pro Lys Gly Asn Ser Gly
                   245                 250                 255
   Glu Pro Gly Ala Pro Gly Ser Lys Gly Asp Thr Gly Ala Lys Gly Glu
               260                 265                 270
   Pro Gly Pro Val Gly Val Gln Gly Pro Pro Gly Pro Ala Gly Glu Glu
           275                 280                 285
   Gly Lys Arg Gly Ala Arg Gly Glu Pro Gly Pro Thr Gly Leu Pro Gly
       290                 295                 300
   Pro Pro Gly Glu Arg Gly Gly Pro Gly Ser Arg Gly Phe Pro Gly Ala
   305                 310                 315                 320
   Asp Gly Val Ala Gly Pro Lys Gly Pro Ala Gly Glu Arg Gly Ser Pro
                   325                 330                 335
   Gly Pro Ala Gly Pro Lys Gly Ser Pro Gly Glu Ala Gly Arg Pro Gly
               340                 345                 350
   Glu Ala Gly Leu Pro Gly Ala Lys Gly Leu Thr Gly Ser Pro Gly Ser
           355                 360                 365
   Pro Gly Pro Asp Gly Lys Thr Gly Pro Pro Gly Pro Ala Gly Gln Asp
       370                 375                 380
   Gly Arg Pro Gly Pro Pro Gly Pro Pro Gly Ala Arg Gly Gln Ala Gly
   385                 390                 395                 400
   Val Met Gly Phe Pro Gly Pro Lys Gly Ala Ala Gly Glu Pro Gly Lys
                   405                 410                 415
   Ala Gly Glu Arg Gly Val Pro Gly Pro Pro Gly Ala Val Gly Pro Ala
               420                 425                 430
   Gly Lys Asp Gly Glu Ala Gly Ala Gln Gly Pro Pro Gly Pro Ala Gly
           435                 440                 445
   Pro Ala Gly Glu Arg Gly Glu Gln Gly Pro Ala Gly Ser Pro Gly Phe
       450                 455                 460
   Gln Gly Leu Pro Gly Pro Ala Gly Pro Pro Gly Glu Ala Gly Lys Pro
   465                 470                 475                 480
   Gly Glu Gln Gly Val Pro Gly Asp Leu Gly Ala Pro Gly Pro Ser Gly
                   485                 490                 495
   Ala Arg Gly Glu Arg Gly Phe Pro Gly Glu Arg Gly Val Gln Gly Pro
               500                 505                 510
   Pro Gly Pro Ala Gly Pro Arg Gly Ala Asn Gly Ala Pro Gly Asn Asp
           515                 520                 525
   Gly Ala Lys Gly Asp Ala Gly Ala Pro Gly Ala Pro Gly Ser Gln Gly
       530                 535                 540
   Ala Pro Gly Leu Gln Gly Met Pro Gly Glu Arg Gly Ala Ala Gly Leu
   545                 550                 555                 560
   Pro Gly Pro Lys Gly Asp Arg Gly Asp Ala Gly Pro Lys Gly Ala Asp
                   565                 570                 575
   Gly Ser Pro Gly Lys Asp Gly Val Arg Gly Leu Thr Gly Pro Ile Gly
               580                 585                 590
   Pro Pro Gly Pro Ala Gly Ala Pro Gly Pro Pro Gly Glu Ser Gly Pro
           595                 600                 605
   Ser Gly Pro Ala Gly Pro Thr Gly Ala Arg Gly Ala Pro Gly Asp Arg
       610                 615                 620
   Gly Glu Pro Gly Pro Pro Gly Pro Ala Gly Phe Ala Gly Pro Pro Gly
   625                 630                 635                 640
   Ala Asp Gly Gln Pro Gly Ala Lys Gly Glu Pro Gly Asp Ala Gly Ala
                   645                 650                 655
   Lys Gly Asp Ala Gly Pro Pro Gly Pro Ala Gly Pro Ala Gly Pro Pro
               660                 665                 670
   Gly Pro Ile Gly Asn Val Gly Ala Pro Gly Ala Lys Gly Ala Arg Gly
           675                 680                 685
   Ser Ala Gly Pro Pro Gly Ala Thr Gly Phe Pro Gly Ala Ala Gly Arg
       690                 695                 700
   Val Gly Pro Pro Gly Pro Ser Gly Asn Ala Gly Pro Pro Gly Pro Pro
   705                 710                 715                 720
   Gly Pro Ala Gly Lys Glu Gly Gly Lys Gly Pro Arg Gly Glu Thr Gly
                   725                 730                 735
   Pro Ala Gly Arg Pro Gly Glu Val Gly Pro Pro Gly Pro Pro Gly Pro
               740                 745                 750
   Ala Gly Glu Lys Gly Ser Pro Gly Ala Asp Gly Pro Ala Gly Ala Pro
           755                 760                 765
   Gly Thr Pro Gly Pro Gln Gly Ile Ala Gly Gln Arg Gly Val Val Gly
       770                 775                 780
   Leu Pro Gly Gln Arg Gly Glu Arg Gly Phe Pro Gly Leu Pro Gly Pro
   785                 790                 795                 800
   Ser Gly Glu Pro Gly Lys Gln Gly Pro Ser Gly Ala Ser Gly Glu Arg
                   805                 810                 815
   Gly Pro Pro Gly Pro Met Gly Pro Pro Gly Leu Ala Gly Pro Pro Gly
               820                 825                 830
   Glu Ser Gly Arg Glu Gly Ala Pro Gly Ala Glu Gly Ser Pro Gly Arg
           835                 840                 845
   Asp Gly Ser Pro Gly Ala Lys Gly Asp Arg Gly Glu Thr Gly Pro Ala
       850                 855                 860
   Gly Pro Pro Gly Ala Pro Gly Ala Pro Gly Ala Pro Gly Pro Val Gly
   865                 870                 875                 880
   Pro Ala Gly Lys Ser Gly Asp Arg Gly Glu Thr Gly Pro Ala Gly Pro
                   885                 890                 895
   Ala Gly Pro Val Gly Pro Ala Gly Ala Arg Gly Pro Ala Gly Pro Gln
               900                 905                 910
   Gly Pro Arg Gly Asp Lys Gly Glu Thr Gly Glu Gln Gly Asp Arg Gly
           915                 920                 925
   Ile Lys Gly His Arg Gly Phe Ser Gly Leu Gln Gly Pro Pro Gly Pro
       930                 935                 940
   Pro Gly Ser Pro Gly Glu Gln Gly Pro Ser Gly Ala Ser Gly Pro Ala
   945                 950                 955                 960
   Gly Pro Arg Gly Pro Pro Gly Ser Ala Gly Ala Pro Gly Lys Asp Gly
                   965                 970                 975
   Leu Asn Gly Leu Pro Gly Pro Ile Gly Pro Pro Gly Pro Arg Gly Arg
               980                 985                 990
   Thr Gly Asp Ala Gly Pro Val Gly  Pro Pro Gly Pro Pro  Gly Pro Pro
           995                 1000                1005
   Gly Pro  Pro Gly Pro Pro
       1010
<210> 12
   <211> 1014
   <212> PRT
   <213> Homo sapiens
<400> 12
   Gly Pro Met Gly Pro Ser Gly Pro Arg Gly Leu Pro Gly Pro Pro Gly
   1               5                   10                  15
   Ala Pro Gly Pro Gln Gly Phe Gln Gly Pro Pro Gly Glu Pro Gly Glu
               20                  25                  30
   Pro Gly Ala Ser Gly Pro Met Gly Pro Pro Gly Pro Pro Gly Pro Pro
           35                  40                  45
   Gly Glu Asn Gly Asp Asp Gly Glu Ala Gly Glu Pro Gly Arg Pro Gly
       50                  55                  60
   Glu Arg Gly Pro Pro Gly Pro Gln Gly Ala Arg Gly Leu Pro Gly Thr
   65                  70                  75                  80
   Ala Gly Leu Pro Gly Met Glu Gly His Arg Gly Phe Ser Gly Leu Asp
                   85                  90                  95
   Gly Ala Glu Gly Asp Ala Gly Pro Ala Gly Pro Glu Gly Glu Pro Gly
               100                 105                 110
   Ser Pro Gly Glu Asn Gly Ala Pro Gly Gln Met Gly Pro Pro Gly Leu
   115 120 125
   Pro Gly Glu Arg Gly Arg Pro Gly Ala Pro Gly Pro Ala Gly Ala Arg
   130 135 140
   Gly Asn Asp Gly Ala Thr Gly Ala Ala Gly Pro Pro Gly Pro Thr Gly
   145 150 155 160
   Pro Ala Gly Pro Pro Gly Phe Pro Gly Ala Val Gly Ala Glu Gly Glu
   165 170 175
   Ala Gly Pro Gln Gly Pro Arg Gly Ser Glu Gly Pro Gln Gly Val Arg
   180 185 190
   Gly Glu Pro Gly Pro Pro Gly Pro Ala Gly Ala Ala Gly Pro Ala Gly
   195 200 205
   Asn Pro Gly Ala Asp Gly Gln Pro Gly Ala Glu Gly Ala Asn Gly Ala
   210 215 220
   Pro Gly Ile Ala Gly Ala Pro Gly Phe Pro Gly Ala Arg Gly Pro Ser
   225 230 235 240
   Gly Pro Gln Gly Pro Gly Gly Pro Pro Gly Pro Glu Gly Asn Ser Gly
   245 250 255
   Glu Pro Gly Ala Pro Gly Ser Glu Gly Asp Thr Gly Ala Glu Gly Glu
   260 265 270
   Pro Gly Pro Val Gly Val Gln Gly Pro Pro Gly Pro Ala Gly Glu Glu
   275 280 285
   Gly Glu Arg Gly Ala Arg Gly Glu Pro Gly Pro Thr Gly Leu Pro Gly
   290 295 300
   Pro Pro Gly Glu Arg Gly Gly Pro Gly Ser Arg Gly Phe Pro Gly Ala
   305 310 315 320
   Asp Gly Val Ala Gly Pro Glu Gly Pro Ala Gly Glu Arg Gly Ser Pro
   325 330 335
   Gly Pro Ala Gly Pro Glu Gly Ser Pro Gly Glu Ala Gly Arg Pro Gly
   340 345 350
   Glu Ala Gly Leu Pro Gly Ala Glu Gly Leu Thr Gly Ser Pro Gly Ser
   355 360 365
   Pro Gly Pro Asp Gly Glu Thr Gly Pro Pro Gly Pro Ala Gly Gln Asp
   370 375 380
   Gly Arg Pro Gly Pro Pro Gly Pro Pro Gly Ala Arg Gly Gln Ala Gly
   385 390 395 400
   Pro Pro Gly Phe Pro Gly Pro Glu Gly Ala Ala Gly Glu Pro Gly Glu
   405 410 415
   Ala Gly Glu Arg Gly Val Pro Gly Pro Pro Gly Ala Val Gly Pro Ala
   420 425 430
   Gly Glu Asp Gly Glu Ala Gly Ala Gln Gly Pro Pro Gly Pro Ala Gly
   435 440 445
   Pro Ala Gly Glu Arg Gly Glu Gln Gly Pro Ala Gly Ser Pro Gly Phe
   450 455 460
   Gln Gly Leu Pro Gly Pro Ala Gly Pro Pro Gly Glu Ala Gly Glu Pro
   465 470 475 480
   Gly Glu Gln Gly Val Pro Gly Asp Leu Gly Ala Pro Gly Pro Ser Gly
   485 490 495
   Ala Arg Gly Glu Arg Gly Phe Pro Gly Glu Arg Gly Val Gln Gly Pro
   500 505 510
   Pro Gly Pro Ala Gly Pro Arg Gly Ala Asn Gly Ala Pro Gly Asn Asp
   515 520 525
   Gly Ala Glu Gly Asp Ala Gly Ala Pro Gly Ala Pro Gly Ser Gln Gly
   530 535 540
   Ala Pro Gly Leu Gln Gly Met Pro Gly Glu Arg Gly Ala Ala Gly Leu
   545 550 555 560
   Pro Gly Pro Glu Gly Asp Arg Gly Asp Ala Gly Pro Glu Gly Ala Asp
   565 570 575
   Gly Ser Pro Gly Glu Asp Gly Val Arg Gly Leu Thr Gly Pro Ile Gly
   580 585 590
   Pro Pro Gly Pro Ala Gly Ala Pro Gly Pro Pro Gly Glu Ser Gly Pro
   595 600 605
   Ser Gly Pro Ala Gly Pro Thr Gly Ala Arg Gly Ala Pro Gly Asp Arg
   610 615 620
   Gly Glu Pro Gly Pro Pro Gly Pro Ala Gly Phe Ala Gly Pro Pro Gly
   625 630 635 640
   Ala Asp Gly Gln Pro Gly Ala Glu Gly Glu Pro Gly Asp Ala Gly Ala
   645 650 655
   Glu Gly Asp Ala Gly Pro Pro Gly Pro Ala Gly Pro Ala Gly Pro Pro
   660 665 670
   Gly Pro Ile Gly Pro Pro Gly Ala Pro Gly Ala Glu Gly Ala Arg Gly
   675 680 685
   Ser Ala Gly Pro Pro Gly Ala Thr Gly Phe Pro Gly Ala Ala Gly Arg
   690 695 700
   Val Gly Pro Pro Gly Pro Ser Gly Asn Ala Gly Pro Pro Gly Pro Pro
   705 710 715 720
   Gly Pro Ala Gly Glu Glu Gly Gly Glu Gly Pro Arg Gly Glu Thr Gly
   725 730 735
   Pro Ala Gly Arg Pro Gly Glu Val Gly Pro Pro Gly Pro Pro Gly Pro
   740 745 750
   Ala Gly Glu Glu Gly Ser Pro Gly Ala Asp Gly Pro Ala Gly Ala Pro
   755 760 765
   Gly Thr Pro Gly Pro Gln Gly Ile Ala Gly Gln Arg Gly Val Val Gly
   770 775 780
   Leu Pro Gly Gln Arg Gly Glu Arg Gly Phe Pro Gly Leu Pro Gly Pro
   785 790 795 800
   Ser Gly Glu Pro Gly Glu Gln Gly Pro Ser Gly Ala Ser Gly Glu Arg
   805 810 815
   Gly Pro Pro Gly Pro Met Gly Pro Pro Gly Leu Ala Gly Pro Pro Gly
   820 825 830
   Glu Ser Gly Arg Glu Gly Ala Pro Gly Ala Glu Gly Ser Pro Gly Arg
   835 840 845
   Asp Gly Ser Pro Gly Ala Glu Gly Asp Arg Gly Glu Thr Gly Pro Ala
   850 855 860
   Gly Pro Pro Gly Ala Pro Gly Ala Pro Gly Ala Pro Gly Pro Val Gly
   865 870 875 880
   Pro Ala Gly Glu Ser Gly Asp Arg Gly Glu Thr Gly Pro Ala Gly Pro
   885 890 895
   Ala Gly Pro Val Gly Pro Ala Gly Ala Arg Gly Pro Ala Gly Pro Gln
   900 905 910
   Gly Pro Arg Gly Asp Glu Gly Glu Thr Gly Glu Gln Gly Asp Arg Gly
   915 920 925
   Ile Glu Gly His Arg Gly Phe Ser Gly Leu Gln Gly Pro Pro Gly Pro
   930 935 940
   Pro Gly Ser Pro Gly Glu Gln Gly Pro Ser Gly Ala Ser Gly Pro Ala
   945 950 955 960
   Gly Pro Arg Gly Pro Pro Gly Ser Ala Gly Ala Pro Gly Glu Asp Gly
   965 970 975
   Leu Asn Gly Leu Pro Gly Pro Ile Gly Pro Pro Gly Pro Arg Gly Arg
   980 985 990
   Thr Gly Asp Ala Gly Pro Val Gly Pro Pro Gly Pro Pro Gly Pro Pro
   995 1000 1005
   Gly Pro Pro Gly Pro Pro
   1010
<210> 13
   <211> 1014
   <212> PRT
   <213> Homo sapiens
<400> 13
   Gly Pro Met Gly Pro Ser Gly Pro Arg Gly Leu Pro Gly Pro Pro Gly
   1               5                   10                  15
   Ala Pro Gly Pro Gln Gly Phe Gln Gly Pro Pro Gly Glu Pro Gly Glu
               20                  25                  30
   Pro Gly Ala Ser Gly Pro Met Gly Pro Arg Gly Pro Pro Gly Pro Pro
           35                  40                  45
   Gly Lys Asn Gly Asp Asp Gly Glu Ala Gly Lys Pro Gly Arg Pro Gly
       50                  55                  60
   Glu Arg Gly Pro Pro Gly Pro Gln Gly Ala Arg Gly Leu Pro Gly Thr
   65                  70                  75                  80
   Ala Gly Leu Pro Gly Met Lys Gly His Arg Gly Phe Ser Gly Leu Asp
                   85                  90                  95
   Gly Ala Lys Gly Asp Ala Gly Pro Ala Gly Pro Lys Gly Glu Pro Gly
               100                 105                 110
   Ser Pro Gly Glu Asn Gly Ala Pro Gly Gln Met Gly Pro Arg Gly Leu
           115                 120                 125
   Pro Gly Glu Arg Gly Arg Pro Gly Ala Pro Gly Pro Ala Gly Ala Arg
       130                 135                 140
   Gly Asn Asp Gly Ala Thr Gly Ala Ala Gly Pro Pro Gly Pro Thr Gly
   145                 150                 155                 160
   Pro Ala Gly Pro Pro Gly Phe Pro Gly Ala Val Gly Ala Lys Gly Glu
                   165                 170                 175
   Ala Gly Pro Gln Gly Pro Arg Gly Ser Glu Gly Pro Gln Gly Val Arg
               180                 185                 190
   Gly Glu Pro Gly Pro Pro Gly Pro Ala Gly Ala Ala Gly Pro Ala Gly
           195                 200                 205
   Asn Pro Gly Ala Asp Gly Gln Pro Gly Ala Lys Gly Ala Asn Gly Ala
       210                 215                 220
   Pro Gly Ile Ala Gly Ala Pro Gly Phe Pro Gly Ala Arg Gly Pro Ser
   225                 230                 235                 240
   Gly Pro Gln Gly Pro Gly Gly Pro Pro Gly Pro Lys Gly Asn Ser Gly
                   245                 250                 255
   Glu Pro Gly Ala Pro Gly Ser Lys Gly Asp Thr Gly Ala Lys Gly Glu
               260                 265                 270
   Pro Gly Pro Val Gly Val Gln Gly Pro Pro Gly Pro Ala Gly Glu Glu
           275                 280                 285
   Gly Lys Arg Gly Ala Arg Gly Glu Pro Gly Pro Thr Gly Leu Pro Gly
       290                 295                 300
   Pro Pro Gly Glu Arg Gly Gly Pro Gly Ser Arg Gly Phe Pro Gly Ala
   305                 310                 315                 320
   Asp Gly Val Ala Gly Pro Lys Gly Pro Ala Gly Glu Arg Gly Ser Pro
                   325                 330                 335
   Gly Pro Ala Gly Pro Lys Gly Ser Pro Gly Glu Ala Gly Arg Pro Gly
               340                 345                 350
   Glu Ala Gly Leu Pro Gly Ala Lys Gly Leu Thr Gly Ser Pro Gly Ser
           355                 360                 365
   Pro Gly Pro Asp Gly Lys Thr Gly Pro Pro Gly Pro Ala Gly Gln Asp
       370                 375                 380
   Gly Arg Pro Gly Pro Pro Gly Pro Pro Gly Ala Arg Gly Gln Ala Gly
   385                 390                 395                 400
   Pro Pro Gly Phe Pro Gly Pro Lys Gly Ala Ala Gly Glu Pro Gly Lys
                   405                 410                 415
   Ala Gly Glu Arg Gly Val Pro Gly Pro Pro Gly Ala Val Gly Pro Ala
               420                 425                 430
   Gly Lys Asp Gly Glu Ala Gly Ala Gln Gly Pro Pro Gly Pro Ala Gly
           435                 440                 445
   Pro Ala Gly Glu Arg Gly Glu Gln Gly Pro Ala Gly Ser Pro Gly Phe
       450                 455                 460
   Gln Gly Leu Pro Gly Pro Ala Gly Pro Pro Gly Glu Ala Gly Lys Pro
   465                 470                 475                 480
   Gly Glu Gln Gly Val Pro Gly Asp Leu Gly Ala Pro Gly Pro Ser Gly
                   485                 490                 495
   Ala Arg Gly Glu Arg Gly Phe Pro Gly Glu Arg Gly Val Gln Gly Pro
               500                 505                 510
   Pro Gly Pro Ala Gly Pro Arg Gly Ala Asn Gly Ala Pro Gly Asn Asp
           515                 520                 525
   Gly Ala Lys Gly Asp Ala Gly Ala Pro Gly Ala Pro Gly Ser Gln Gly
       530                 535                 540
   Ala Pro Gly Leu Gln Gly Met Pro Gly Glu Arg Gly Ala Ala Gly Leu
   545                 550                 555                 560
   Pro Gly Pro Lys Gly Asp Arg Gly Asp Ala Gly Pro Lys Gly Ala Asp
                   565                 570                 575
   Gly Ser Pro Gly Lys Asp Gly Val Arg Gly Leu Thr Gly Pro Ile Gly
               580                 585                 590
   Pro Pro Gly Pro Ala Gly Ala Pro Gly Asp Lys Gly Glu Ser Gly Pro
           595                 600                 605
   Ser Gly Pro Ala Gly Pro Thr Gly Ala Arg Gly Ala Pro Gly Asp Arg
       610                 615                 620
   Gly Glu Pro Gly Pro Pro Gly Pro Ala Gly Phe Ala Gly Pro Pro Gly
   625                 630                 635                 640
   Ala Asp Gly Gln Pro Gly Ala Lys Gly Glu Pro Gly Asp Ala Gly Ala
                   645                 650                 655
   Lys Gly Asp Ala Gly Pro Pro Gly Pro Ala Gly Pro Ala Gly Pro Pro
               660                 665                 670
   Gly Pro Ile Gly Asn Val Gly Ala Pro Gly Ala Lys Gly Ala Arg Gly
           675                 680                 685
   Ser Ala Gly Pro Pro Gly Ala Thr Gly Phe Pro Gly Ala Ala Gly Arg
       690                 695                 700
   Val Gly Pro Pro Gly Pro Ser Gly Asn Ala Gly Pro Pro Gly Pro Pro
   705                 710                 715                 720
   Gly Pro Ala Gly Lys Glu Gly Gly Lys Gly Pro Arg Gly Glu Thr Gly
                   725                 730                 735
   Pro Ala Gly Arg Pro Gly Glu Val Gly Pro Pro Gly Pro Pro Gly Pro
               740                 745                 750
   Ala Gly Glu Lys Gly Ser Pro Gly Ala Asp Gly Pro Ala Gly Ala Pro
           755                 760                 765
   Gly Thr Pro Gly Pro Gln Gly Ile Ala Gly Gln Arg Gly Val Val Gly
       770                 775                 780
   Leu Pro Gly Gln Arg Gly Glu Arg Gly Phe Pro Gly Leu Pro Gly Pro
   785                 790                 795                 800
   Ser Gly Glu Pro Gly Lys Gln Gly Pro Ser Gly Ala Ser Gly Glu Arg
                   805                 810                 815
   Gly Pro Pro Gly Pro Met Gly Pro Pro Gly Leu Ala Gly Pro Pro Gly
               820                 825                 830
   Glu Ser Gly Arg Glu Gly Ala Pro Gly Ala Glu Gly Ser Pro Gly Arg
           835                 840                 845
   Asp Gly Ser Pro Gly Ala Lys Gly Asp Arg Gly Glu Thr Gly Pro Ala
       850                 855                 860
   Gly Pro Pro Gly Ala Pro Gly Ala Pro Gly Ala Pro Gly Pro Val Gly
   865                 870                 875                 880
   Pro Ala Gly Lys Ser Gly Asp Arg Gly Glu Thr Gly Pro Ala Gly Pro
                   885                 890                 895
   Ala Gly Pro Val Gly Pro Ala Gly Ala Arg Gly Pro Ala Gly Pro Gln
               900                 905                 910
   Gly Pro Arg Gly Asp Lys Gly Glu Thr Gly Glu Gln Gly Asp Arg Gly
           915                 920                 925
   Ile Lys Gly His Arg Gly Phe Ser Gly Leu Gln Gly Pro Pro Gly Pro
       930                 935                 940
   Pro Gly Ser Pro Gly Glu Gln Gly Pro Ser Gly Ala Ser Gly Pro Ala
   945                 950                 955                 960
   Gly Pro Arg Gly Pro Pro Gly Ser Ala Gly Ala Pro Gly Lys Asp Gly
                   965                 970                 975
   Leu Asn Gly Leu Pro Gly Pro Ile Gly Pro Pro Gly Pro Arg Gly Arg
   980 985 990
   Thr Gly Asp Ala Gly Pro Val Gly Pro Pro Gly Pro Pro Gly Pro Pro
   995 1000 1005
   Gly Pro Pro Gly Pro Pro
   1010

## Description

### FIELD OF THE INVENTION

The invention relates to recombinant gelatins, and to recombinant gelatins useful in gelatin capsule manufacture, and to compositions, gelatin capsules, and gelatin films comprising these, as well as methods of production.

### BACKGROUND OF THE INVENTION

Gelatin is typically manufactured from naturally occurring collagen through processing of animal hides and bones, most often from bovine and porcine sources. Due to variability in collagen source material and methods of extraction and processing, the physiochemical properties of such animal-derived gelatin preparations exhibit significant variability in characteristics and, correspondingly, in performance. Gelatin extracted from animal sources is thus a mixture of heterogeneous proteins, containing polypeptide fragments of varying molecular weights, melting profiles, gelling properties, etc.

The unique properties of gelatin make it a component essential to the manufacturing and performance of capsules and related products. Over 40,000 metric tons of gelatin are used per year in the manufacturing of soft gelatin and hard gelatin (or hard shell) capsules alone. The structure and composition of gelatin allow formation of a thermally reversible material that can be a solid at room temperature and melt at physiologically relevant temperatures, and non-gelatin replacement materials have failed to provide comparable performance.

Manufacture of gelatin capsules is an extensive process. Characteristics of gelatin such as thermoreversibility (e.g., melting temperature, gelling temperature, etc.) are critical to high-speed capsule manufacturing and in determining product composition, process predictability, and reproducibility. Heterogeneity of the gelatin material can lead to a gelatin product encompassing different fractions or portions of gelatins having varying characteristics, i.e., ranges of molecular weights, melting temperatures, etc. Properties of gelatin such as viscosity, gel strength, surface effects, foaming properties, film formation, and interaction with plastizicers, water, and pigments, need to be carefully monitored in order to limit variability in performance throughout the manufacturing process, and through storage and downstream use of the gelatin-containing product.

To insure predictable behavior, and minimize variation in manufacturing process and outcome, capsule manufacturers currently resort to extensive testing and mixing of different lots of various commercially available animal-derived gelatins in order to produce a resultant gelatin material having physical characteristics that range within acceptable parameters. Additionally, there are limits to the extent to which the physical/performance properties of currently available animal-source gelatins can be altered or controlled.

Gelatin capsule manufacture involves the use of high-temperature solutions of gelatin over long periods of time. Gelatin maintained under such conditions hydrolyses and its Bloom strength and viscosity decrease, thereby affecting the consistency and performance of the capsule manufacturing process. Manufacturers typically use small batches of gelatin solutions for capsule manufacturing to ensure a quick turnover of material and reduce the decline in physical properties of the gelatin.

Inconsistency or variability in the gelatin structure can affect downstream capsule performance once the capsule is filled and/or administered. Upon capsule formation, gelatin structure and resulting capsule performance may be altered by, for example, crosslinking, occurring as a result of storage conditions, compromising the stability, longevity, and usability of the product capsule. Gelatin crosslinking can result in the formation of an insoluble film inside the capsule that will lead to dissolution variability, increasing the cost of manufacturing by necessitating expensive stability and bio-dissolution studies, and compromising consistency of performance. The likelihood of crosslinking is related to the composition and varying physical properties of the gelatin mixture used in capsule manufacture. Variability in gelatin composition can also affect the brittleness of hard shell capsules.

In summary, there is a need in the art for a material useful in the manufacture of soft gelatin and hard gelatin capsules that provides consistent, reproducible, and fully controlled performance. The present invention meets this need by providing recombinant gelatins having specific, uniform, reproducible, and controlled characteristics such as, for example, homogeneity, uniform molecular weight, specific Bloom and gel strength, viscosity, melting temperature, thermoreversibility, thermoplasticity, thermoelasticity, etc., optimal for capsule manufacture and performance.

In summary, there is a need in the art for a material useful in the manufacture of soft gelatin and hard gelatin capsules that provides consistent, reproducible, and fully controlled performance. In particular, there is a need in the art for a material useful for capsule manufacture that is resistant to the undesirable changes in its physical properties, such as, for example, hydrolysis and cross-linking, as well as decreased Bloom strength and viscosity, etc. The present invention meets this need by providing gelatin materials having specific, uniform, reproducible, and controlled characteristics such as, for example, homogeneity, single molecular weight species, specific Bloom and gel strength, viscosity, melting temperature, thermoreversibility, thermoplasticity, thermoelasticity, etc., optimal for capsule manufacture and performance. The present invention further meets this need by providing gelatin materials and formulations useful for capsule manufacturing, such materials and formulations having thermal stability and a stable viscosity.

### SUMMARY OF THE INVENTION

The invention relates to recombinant gelatins, and to recombinant gelatins useful in gelatin capsule manufacture, and to compositions, gelatin capsules, and gelatin films comprising these, as well as methods of production.

In one aspect, the invention discloses a single molecular species recombinant gelatin useful for capsule manufacture, wherein the viscosity of a 20% solution comprising the single molecular species recombinant gelatin decreases less than about 20% when the 20% solution is maintained for 17 hours at 60°C. In another aspect, the invention discloses a single molecular species recombinant gelatin useful for capsule manufacture, wherein the viscosity of a 20% solution comprising the single molecular species recombinant gelatin decreases less than about 15% when the 20% solution is maintained for 17 hours at 60°C. A single molecular species recombinant gelatin useful for capsule manufacture, wherein the viscosity of a 20% solution comprising the single molecular species recombinant gelatin decreases less than about 10% when the 20% solution is maintained for 17 hours at 60°C, is also provided.

In various embodiments, the invention discloses a single molecular species recombinant gelatin useful for capsule manufacture, wherein the viscosity of a 20% solution comprising the single molecular species recombinant gelatin decreases less than about 5% when the 20% solution is maintained for 17 hours at 60°C; and a single molecular species recombinant gelatin useful for capsule manufacture, wherein the viscosity of a 20% solution comprising the single molecular species recombinant gelatin decreases about 3.5% when the 20% solution is maintained for 17 hours at 60°C.

In certain aspects, the invention discloses a single molecular species recombinant gelatin useful for capsule manufacture, wherein the viscosity of a 20% solution comprising the single molecular species recombinant gelatin is about 42.5 centipoises at 60°C; and a single molecular species recombinant gelatin useful for capsule manufacture, wherein the viscosity of a 20% solution comprising the single molecular species recombinant gelatin is about 41.0 centipoises when the 20% solution is maintained for 17 hours at 60°C.

The invention discloses a composition useful for capsule manufacture, the composition comprising a single molecular species recombinant gelatin and at least one capsule manufacturing agent. In various embodiments, the capsule manufacturing agent is selected from the group consisting of a plasticizer, a coloring agent, an opacifying agent, a flavoring, a sugar, an acid, and a medicament. In a particular embodiment, the capsule manufacturing agent is a plasticizer. In another embodiment, the capsule manufacturing agent is a plasticizer, and the composition further comprises a coloring agent. In various embodiments, the coloring agent is selected from the group consisting of an F D & C dye and a D & C dye. In some embodiments, the composition further comprises a second single molecular species recombinant gelatin.

In one aspect, the invention discloses a composition useful in capsule manufacture, the composition comprising a gelatin component, wherein the gelatin component consists of a single molecular species recombinant gelatin. In a further aspect, the invention discloses a composition useful in capsule manufacture, the composition comprising a gelatin component, wherein the gelatin component consists of a first and a second single molecular species recombinant gelatin. In another aspect, the invention discloses a composition useful in capsule manufacture, the composition comprising a gelatin component, wherein the gelatin component consists of multiple single molecular species recombinant gelatins.

In another aspect, such compositions further comprise a capsule manufacturing agent. In various aspects, the capsule manufacturing agent is selected from the group consisting of a plasticizer, a coloring agent, an opacifying agent, a flavoring, a sugar, an acid, and a medicament.

The invention discloses in one embodiment a single molecular species recombinant gelatin, wherein a gelatin film comprising the single molecular species recombinant gelatin, and having a thickness of about 0.0030 inches, and having a length of about 10 cm and a width of about 2 cm, has a force at break of less than about 13 Newton. In another embodiment, the invention discloses a single molecular species recombinant gelatin, wherein a gelatin film comprising the single molecular species recombinant gelatin, and having a thickness of about 0.0030 inches, and having a length of about 10 cm and a width of about 2 cm, has a force at break of about 11.6 Newton. In a further embodiment, the invention discloses a single molecular species recombinant gelatin, wherein a gelatin film comprising the single molecular species recombinant gelatin, and having a thickness of about 0.0030 inches, and having a length of about 10 cm and a width of about 2 cm, has a greater than about 300 % elongation at break.

Gelatin films comprising the single molecular species gelatins of the present invention are also disclosed. In one aspect, the invention discloses a gelatin film comprising a single molecular species recombinant gelatin, wherein the gelatin film has a thickness of about 0.0030 inches, and wherein a 10 cm long and 2 cm wide strip of the gelatin film has a force at break of less than about 13 Newton. In another aspect, the invention discloses a gelatin film comprising a single molecular species recombinant gelatin, wherein the gelatin film has a thickness of about 0.0030 inches, and wherein a 10 cm long and 2 cm wide strip of the gelatin film has a force at break of about 11.6 Newton. In a certain aspect, the invention discloses a gelatin film comprising a single molecular species recombinant gelatin, wherein the gelatin film has a thickness of about 0.0030 inches, and wherein a 10 cm long and 2 cm wide strip of the gelatin film has a greater than about 300 % elongation at break.

A single molecular species recombinant gelatin useful for capsule manufacture, wherein the single molecular species gelatin is selected from the group consisting of SEQ ID NO:1, 2, 3, 4, 5, 6, 7, 8, 9, 10, 11, 12, and 13, is provided herein. The invention additionally discloses a gelatin capsule comprising a single molecular species recombinant gelatin useful for capsule manufacture, wherein the gelatin is selected from the group consisting of SEQ ID NO:1, 2, 3, 4, 5, 6, 7, 8, 9, 10, 11, 12, and 13.

Methods for manufacturing a gelatin capsule are provided, the methods comprising providing a mixture comprising a single molecular species recombinant gelatin and a capsule manufacturing agent, and forming a gelatin capsule from the mixture. In particular embodiments, the capsule manufacturing agent is a plasticizer.

Methods for manufacturing a gelatin film, the methods comprising providing a mixture comprising a single molecular species recombinant gelatin and a plasticizer, and forming a gelatin film from the mixture, are also disclosed herein.

### DESCRIPTION OF THE INVENTION

Before the present proteins, nucleotide sequences, and methods are described, it is understood' that this invention is not limited to the particular methodology, protocols, cell lines, vectors, and reagents described, as these may vary. It is also to be understood that the terminology used herein is for the purpose of describing particular embodiments only, and is not intended to limit the scope of the present invention.

It must be noted that as used herein, and in the appended claims, the singular forms "a," "an," and "the" include plural reference unless the context clearly dictates otherwise. Thus, for example, reference to "a single molecular species recombinant gelatin" is reference to one or more of such single molecular species recombinant gelatins and equivalents thereof known to those skilled in the art, and so forth.

The practice of the present invention will employ, unless otherwise indicated, conventional methods of chemistry, biochemistry, molecular biology, immunology and pharmacology, within the skill of the art. Such techniques are explained fully in the literature. See, e.g., Gennaro, A.R., ed. (1990) Remington's Pharmaceutical Sciences, 18th ed., Mack Publishing Co.; Colowick, S. et al., eds., Methods In Enzymology, Academic Press, Inc.; Handbook of Experimental Immunology, Vols. I-IV (D.M. Weir and C.C. Blackwell, eds., 1986, Blackwell Scientific Publications); Maniatis, T. et al., eds. (1989) Molecular Cloning: A Laboratory Manual, 2nd edition, Vols. I-III, Cold Spring Harbor Laboratory Press; Ausubel, F. M. et al., eds. (1999) Short Protocols in Molecular Biology, 4th edition, John Wiley & Sons; Ream et al., eds. (1998) Molecular Biology Techniques: An Intensive Laboratory Course, Academic Press); PCR (Introduction to Biotechniques Series), 2nd ed. (Newton & Graham eds., 1997, Springer Verlag).

### INVENTION

The present invention discloses recombinant gelatins useful in the manufacture of soft gelatin and hard gelatin capsules. In particular, the present invention discloses recombinant gelatins having characteristics optimal for use in the manufacture and performance of capsules, encapsulants, films, etc. The invention further discloses the production and use of recombinant gelatins having desired performance properties, i.e., viscosity, Bloom strength, and thermoreversibility. In particular aspects, the invention discloses recombinant gelatins having specific characteristics, including specific values or ranges of molecular weights, concentrations, melting temperatures, levels of hydroxylation, collagen type derivation, etc.

In one aspect, the present invention discloses a gelatin capsule comprising recombinant gelatin having an amino acid sequence selected from the following: amino acid residue 531 to residue 1030 of human α1(I) collagen (SEQ ID NO:1); amino acid residue 531 to residue 1030 of human α1(1) collagen, wherein Val579 and Met580 are replaced with proline residues (SEQ ID NO:2); amino acid residue 531 to residue 1192 of human α1(I) collagen (SEQ ID NO:3); amino acid residue 531 to residue 1192 of human α1(I) collagen, wherein Val579 and Met580 are replaced with proline residues (SEQ ID NO:4); amino acid residue 179 to residue 1192 of human α1(I) collagen (SEQ ID NO:5); amino acid residue 179 to residue 1192 of human α1(I) collagen, wherein Arg220 and Arg304 are replaced with proline residues (SEQ ID NO:6); amino acid residue 179 to residue 1192 of human α1(I) collagen, wherein Val579 and Met580 are replaced with proline residues (SEQ ID NO:13); and amino acid residue 179 to residue 1192 of human α1(I) collagen, wherein Arg220, Arg304, Val579, and Met580 are replaced with proline residues (SEQ ID NO:7).

In another aspect, the present invention discloses a gelatin capsule comprising recombinant gelatin having an amino acid sequence selected from the following: amino acid residue 179 to residue 1192 of human α1(I) collagen, wherein Arg220, Arg304, Val579, Met580, Asp780, Lys781, Asn855, and Val856 are replaced with proline residues (SEQ ID NO:8); amino acid residue 179 to residue 1192 of human α1(I) collagen, wherein Arg220, Arg304, Asp780, Lys781, Asn855, and Val856 are replaced with proline residues (SEQ ID NO:9); amino acid residue 179 to residue 1192 of human α1(I) collagen, wherein Arg220, Arg304, Asn855, and Val856 are replaced with proline residues (SEQ ID NO:10); amino acid residue 179 to residue 1192 of human α1(1) collagen, wherein Arg220, Arg304, Asp780, and Lys781 are replaced with proline residues (SEQ ID NO:11); and amino acid residue 179 to residue 1192 of human α1(I) collagen, wherein Arg220, Arg304, Val579, Met580, Asp780, Lys781, Asn855, and Val856 are replaced with proline residues and all remaining lysine residues are replaced with glutamic acid residues (SEQ ID NO:12).

In certain aspects, it may be desirable - depending on the material to be encapsulated - to produce a gelatin for use in capsule manufacture, wherein the gelatin is devoid of amino acids, such as lysine, which can be involved in chemical reactions that affect performance of some drugs/medications (e.g., by affecting dissolution or bioavailability of the active agent/drug, etc.). For example, in circumstances in which it may be desirable to have a low isoelectric point, a gelatin comprising a sequence such as SEQ ID NO:12 may be used. In instances in which it is desirable to have a higher isoelectric point, e.g., in order to minimize or eliminate interaction of particular drugs (for example, protease inhibitors, etc.) with the encapsulating gelatin, a gelatin comprising a sequence such as that of SEQ ID NO:12, but with the remaining lysine residues switched to histidine residues rather than glutamic acid residues, may be preferred. It is a clear advantage of the present materials that, unlike prior art materials, they can be deliberately and reproducibly made to possess certain characteristics which optimize the performance of these materials in capsule manufacture, such as by minimizing or eliminating undesirable interactions with the encapsulated material.

Molecular weight distribution of gelatin can be critical to various aspects of capsule manufacture and performance, as molecular weight distribution can influence, for example, the viscosity and/or gel strength of a gelatin sample. The present invention discloses recombinant gelatins of specific and uniform molecular weight for use in the manufacture of soft gelatin and hard gelatin capsules. In certain embodiments, the present invention discloses a gelatin capsule comprising recombinant gelatin, wherein the recombinant gelatin is a single molecular species recombinant gelatin, e.g., a gelatin made up of individual recombinant gelatin polypeptides with specific and uniform characteristics. For example, the recombinant gelatin polypeptides can have a uniform molecular weight. The molecular weight of the recombinant gelatin polypeptides can be, for example, 100 kD, 65 kD, 50 kD, etc. Additionally, the gelatin can be a single molecular species recombinant gelatin, e.g., a gelatin comprising gelatin polypeptides having the same amino acid sequence and the same molecular weight. In various embodiments, the single molecular species recombinant gelatin is selected from the group consisting of SEQ ID NOs: 1, 2, 3, 4, 5, 6, 7, 8, 9, 10, 11, 12, and 13.

The present invention discloses recombinant gelatin having specific and defined viscosity. In general, the viscosity of a gelatin solution increases with increasing gelatin concentration and with decreasing temperature. Additionally, viscosity of a gelatin solution increases with increasing molecular weight of the gelatin components. A high-viscosity gelatin solution could consist, therefore, of a high concentration of low molecular weight gelatins, or of a lower concentration of high molecular weight gelatins. Viscosity also affects gel properties including setting and melting point. High-viscosity gelatin solutions provide gelatins with higher melting and setting points and rates than do lower viscosity gelatin solutions. The present invention provides recombinant gelatins of specific viscosities.

The present invention discloses recombinant gelatins of specific and defined molecular weights and specific and defined viscosities useful in the manufacture of soft gelatin and hard gelatin capsules. For example, in one aspect, the present invention discloses 50 kD recombinant gelatins (6.67% solution) having viscosities of about 3.8, 3.1, 2.6, or 2.1 centipoises (cP) at 30°C, 40°C, 50°C, or 60°C, respectively. In another aspect, the present invention discloses 65 kD recombinant gelatins (6.67% solution) having viscosities of about 4.8, 3.7, 3.1, or 2.6 cP at 30°C, 40°C, 50°C, or 60°C, respectively. In yet another aspect, the present invention provides 100 kD recombinant gelatins (6.67% solution) having viscosities of about 6.6, 5.3, 4.4, or 3.7 cP at 30°C, 40°C, 50°C, or 60°C, respectively.

The present invention also discloses recombinant gelatins obtained from recombinant collagens and having specific and defined viscosities useful in the manufacture of soft gelatin and hard gelatin capsules. In one aspect, the recombinant gelatins are obtained from recombinant collagen type I. In another aspect, the recombinant gelatins are obtained from recombinant collagen type I homotrimers. In certain aspects, viscosity of recombinant gelatins obtained from recombinant collagen is increased or decreased depending on the degree of hydroxylation of the recombinant collagen starting material. For example, in one aspect, the present invention discloses recombinant gelatins derived from non-hydroxylated homotrimeric recombinant human collagen type I (6.67% solution) and having a viscosity of about 7.0, 5.3, 4.3, or 3.6 cP at 30°C, 40°C, 50°C, or 60°C, respectively. In another aspect, the present invention discloses recombinant gelatins derived from partially hydroxylated (e.g., 65% hydroxylated) homotrimeric recombinant human collagen type I (6.67% solution) and having a viscosity of about 6.7, 4.7, or 4.1 cP at 40°C, 50°C, or 60°C, respectively. In yet another aspect, the present invention discloses recombinant gelatins derived from fully hydroxylated homotrimeric recombinant human collagen type I (6.67% solution) and having a viscosity of about 7.0, 5.3, or 4.4 cP at 40°C, 50°C, or 60°C, respectively.

Gelatins of various gel strengths are suitable for various soft gelatin and hard gelatin capsule applications. The firmness or strength of the set gelatin is typically measured by calculating the Bloom value, which can be determined using international standards and methodology. Briefly, the Bloom strength is a measurement of the strength of a gel formed by a 6.67% solution (w/v) of gelatin in a constant temperature bath (10°C) over 17 hours. A standard Texture Analyzer is used to measure the weight in grams required to depress a standard 0.5 inch diameter AOAC (Association of Official Agricultural Chemists) plunger 4 millimeters into the gel. If the weight in grams required for depression of the plunger is 200 grams, the particular gelatin has a Bloom value of 200 g. (See, e.g., United States Pharmacopoeia and Official Methods of Analysis of AOAC International, 17th edition, Volume II.)

The present invention discloses recombinant gelatins of specific molecular weights having specific Bloom or gel strengths useful in the manufacture of soft and hard gelatin capsules. For example, in one aspect, the present invention discloses a 50 kD recombinant gelatin having a Bloom strength of about 10 g. In another aspect, the present invention discloses a 65 kD recombinant gelatin having a Bloom strength of about 145 g. In yet another aspect, the present invention provides a 100 kD recombinant gelatin having a Bloom strength of about 225 g.

In certain aspects, Bloom strength of recombinant gelatins obtained from recombinant collagen is increased or decreased depending on the degree of hydroxylation of the recombinant collagen starting material. For example, in one aspect, the present invention discloses recombinant gelatin derived from nonhydroxylated homotrimeric recombinant human collagen type I and having a Bloom value of about 200 g. In another aspect, the present invention discloses recombinant gelatin derived from partially hydroxylated (e.g., 65% hydroxylated) homotrimeric recombinant human collagen type I and having a Bloom value of about 290 g. In yet another aspect, the present invention discloses recombinant gelatin derived from fully hydroxylated homotrimeric recombinant human collagen type I and having a Bloom value of about 400 g. A recombinant gelatin with a high Bloom strength can be used, for example, in the manufacture of gelatin capsules, and could allow for the manufacture of a lighter and thinner capsule. This is also beneficial in terms of economics, as less material would need to be used to provide a gelatin of sufficient strength.

A unique property of gelatin critical to the manufacture and performance of soft gelatin and hard gelatin capsules is thermoreversibility. As a thermoreversible material, gelatin has the ability to exist interchangeably in liquid or in solid state in a temperature-specific and reversible manner. The thermoreversibility of gelatin is exploited in the manufacture of gelatin capsules. This property of gelatin results in a material that can be malleable and in solution as appropriate during manufacture, allowing for mixture with dyes and other capsule components, and can be transformed into a solid or gelling state upon capsule formation. These parameters are equally critical to gelatin capsule performance, as a gelatin capsule can exist in solid form at ambient transport and storage temperatures, yet, upon administration/ingestion, can melt at body temperatures, thus releasing the capsule's active ingredient. Substitutes for gelatin, e.g., polysaccharides, etc., have failed to mimic these thermoreversible properties, constituting materials that dissolve rather than melt across a temperature gradient, imposing restrictions/limitations on the manufacturing process, as well as resulting in capsules with less stability and elasticity.

The present invention also discloses recombinant gelatins with specific melting temperatures or ranges of melting temperatures useful for the manufacture of soft gelatin and hard gelatin capsules. In one aspect, the present invention discloses a gelatin capsule comprising recombinant gelatin having a defined melting temperature, such as, for example, about 28°C, 29°C, 30°C, 31°C , 32°C , 33°C , 34°C , 35°C, or 36°C. In other aspects, the present invention discloses a gelatin capsule comprising recombinant gelatin having a melting temperature ranging from 28°C to 40°C, having a median Tₘ value of about 34°C to 36°C, or ranging from about 28°C to 36°C, with a median Tₘ value of about 30°C to 32°C.

The invention also discloses recombinant gelatins having a particular gelling point useful for the manufacture of soft gelatin and hard gelatin capsules. In one embodiment, the present invention discloses recombinant gelatins having a setting or gelling point of from 15 to 35°C. In further embodiments, the recombinant gelatins have a setting point of from 15 to 25°C, from 25 to 35°C, and from 20 to 30°C.

One problem associated with the use of standard commercial gelatins is the variability inherent in this material. In order to ensure that gelatin material has the appropriate viscosity, gel strength, etc., for use in capsule manufacture, different lots of gelatin must be analyzed, combined, tested, etc., to determine if their respective properties fall within the appropriate physical parameters. The recombinant gelatins of the present invention thus provide a heretofore unattainable level of control over the physical and performance properties, as gelatins for use in capsule manufacture can be produced reproducibly and in fully characterized fashion, eliminating the need to analyze and blend lots to obtain material within a desired range of specifications, and permitting standardization and exact reproduction of the manufacturing process.

Furthermore, in addition to being exactly reproducible the recombinant gelatins of the present invention offer a powerful new opportunity to use in capsule manufacture a material specifically designed and optimized for that application. In particular, it is known that the unique gelling capabilities of gelatin are related to a number of physical performance parameters inherent to the material, i.e., viscosity, gel strength, etc. These parameters are not readily manipulated in gelatin extracted from animal sources. In addition, determination of the parameters/properties of gelatin for use in capsule manufacture requires extensive testing and analysis prior to manufacturing, further manipulation of the material (e.g., blending lots, etc.) to arrive at usable gelatin, and can even require constant alterations in or additions to the manufacturing process as necessary to account for variations or ranges in the properties of gelatin used at any given time. In contrast, the present invention provides for recombinant gelatins designed for specific performance, i.e., exact and consistent viscosity measurements, gel/Bloom strength values.

For example, viscosity, or resistance to flow in a solution, and gel/Bloom strength, resistance to perturbation of a solid, are features of gelatin critical to capsule manufacture and performance. These parameters are impossible to predict and to define as absolute values in the heterogeneous mixture of protein fragments that constitutes gelatin extracted from animal sources. This variability in starting materials creates a need for extensive analysis and testing of various lots, and blending and retesting of gelatin material, in order to obtain a solution suitable for use. In contrast, the recombinant gelatins of the present invention can be uniformly and reproducibly obtained, having exact specifications leading to consistent and predictable performance.

The present invention discloses the production and use of recombinant gelatins having distinct physical characteristics. In one aspect, the present invention discloses production and use of recombinant gelatins having molecular weights or ranges of molecular weights that render these materials optimal for capsule manufacture and performance. Gelatin currently used in capsule manufacture is a material containing a distribution of varying molecular weights. This variation in molecular weights is an inevitable result of the extraction and denaturation of collagen to produce gelatin, and the lengthy and harsh associated chemical processes. A typical molecular weight distribution will include gelatin molecules corresponding to individual full-length collagen chains, to fragments or portions of these individual chains, and to dimer and trimer complexes. The ability of gelatin to gel or set, having thermoreversible properties that allow gelatin to exist movably along a solution/solid gradient, is due to the interactions of the individual gelatin molecules, as interchain bonds are formed, disturbed, and reformed in response to surrounding environmental forces. Interchain interactions between gelatin molecules, and the degree to which they occur, in part, dictates the stability, viscosity, gel strength, melting temperature, etc., of gelatin.

The present recombinant gelatins provide unexpected and previously unavailable advantages. In particular, in one aspect the present invention discloses a homogeneous recombinant gelatin, one with conformity of individual gelatin polypeptides. This homogeneous material optimizes interchain interactions by allowing for better alignment and more cohesive association of individual gelatin polypeptides. This increased stability of the gelatin meshwork leads to predictability in and reversibility of the gelatin phases, eliminating the need for pre-manufacturing analysis of gelatin and for batch-by-batch modification/variation of the manufacturing process itself.

For extracted gelatins, measurements such as molecular weights, gel strengths, and viscosity are assigned as average or median values encompassing overall a material with fractional variation in physical parameters. In contrast, the present invention provides recombinant gelatins having specific and absolute molecular weights, viscosities, and Bloom strengths, etc., representing exact and fixed values that can be reproducibly obtained and that are consistent throughout a particular gelatin. (See, e.g., Tables 1 and 2, below).

The present invention provides compositions and formulations useful for capsule manufacture. Compositions and formulations useful for capsule manufacture can include recombinant gelatin, such as single molecular species recombinant gelatin, and other ingredients, such as, for example, a plasticizer, water, etc., and may contain additional ingredients such as, for example, preservatives, coloring agents (e.g., dyes), opacifying agents (e.g., titanium dioxide), flavorings (e.g., ethyl vanillin), sugars (e.g., sucrose), acids (e.g., fumaric acid), and medicaments to achieve desired effects. Commonly used plasticizers in capsule compositions and formulations include, for example, glycerin, glycerol, sorbitol, sorbitan, polyhydric alcohols and hygroscopic polyols, natural gums, sugars, etc. The ratio by weight of dry plasticizers to dry gelatin determines the hardness of the gelatin capsule. For example, a hard gelatin capsule would have a ratio by weight of dry plasticizer to dry gelatin of about 0.4:1; a medium gelatin capsule would have a ratio by weight of dry plasticizer to dry gelatin of about 0.6:1; and a soft gelatin capsule would have a ratio by weight of dry plasticizer to dry gelatin of about 0.8:1. Commonly used coloring agents in capsule compositions and formulations include, for example, various dyes, such as FD&C and D&C water-soluble dyes, certified lakes, pigments, and vegetable colors, used alone or in combination.

The present recombinant gelatins provide advantages in the context of transport and storage of capsules prior to use, improving the lifetime of the capsules and expanding the environmental and temporal conditions under which the capsules can be stored, while still provide optimal performance. Further, the process by which extracted gelatin is obtained can lead to, or fail to remove, various reactive groups, e.g., disulfides, aldehydes, other groups that might form through oxidation during the manufacturing process, etc., that can potentially result in reactions between the gelatin capsule and the fill material. In one aspect, the invention discloses recombinant gelatins having specific modifications that reduce or eliminate any interactions of the capsule gelatin with the contents of the capsule, such as fill, drug, etc. Such interactions, including disulfide bond formation between gelatin and the fill material, which leads to undesirable effects on both capsule performance and drug stability. In one aspect, the present invention discloses recombinant gelatins having reduced ability to form disulfide bonds, including, for example, recombinant gelatins lacking cysteine residues. Recombinant gelatins of the present invention are also free of other chemical alterations or oxidative byproducts, such as, for example, aldehydes, which can result from the harsh and lengthy process of producing extracted gelatins.

The recombinant gelatins of the present invention, or recombinant collagens from which the present recombinant gelatins are derived, can be subject to various post-translational modifications. Different post-translational modifications, and different post-translational enzymes, e.g., prolyl hydroxylase, lysyl hydroxylase, etc., can effect, for example, Bloom strength, viscosity, melting temperature, thermoreversibility, and other physical characteristics of the present recombinant gelatins. In one aspect, recombinant gelatins having a particular degree of proline hydroxylation, i.e., gelatin that is nonhydroxylated, partially hydroxylated, and fully hydroxylated. The degree of hydroxylation can be chosen to enhance or achieve certain effects on various properties of the present recombinant gelatins, e.g., thermoreversibility, Bloom strength, viscosity, melting temperature, etc. Such recombinant gelatins could be used directly in gelatin capsule manufacture, without any fractionation or separation of differently hydroxylated portions.

The present invention also discloses recombinant gelatins that are crosslinked to varying degrees, as desired to obtain particular results. In one aspect, the present recombinant gelatins are crosslinked to produce higher molecular weight gelatins useful in the manufacture of soft gelatin and hard gelatin capsules. Crosslinking can be achieved by different methods, such as by biological or chemical modification. For example, in one embodiment, recombinant gelatin or a polypeptide from which gelatin can be derived is expressed in the presence of lysyl hydroxylase and lysyl oxidase. In another embodiment, the polypeptide is modified by crosslinking after expression. In a further aspect, the present invention provides for imposition of crosslinking by chemical means, such as by reactive chemical crosslinkers, for example 1-ethyl-3-(dimethylaminopropyl) carbodiimide hydrochloride (EDC). Other chemical cross-linking agents, such as bis(sulfosuccinimidyl) suberate (BS³), 3,3'-dithiobis(sulfosuccinimidyl) propionate (DTSSP), and Tris-sulfosuccinimidyl aminotriacetate (Sulfo-TSAT) may also be used, as can various agents known in the art. Additionally, the present invention provides recombinant gelatins with varying degrees of crosslinking, useful for obtaining recombinant gelatins of desired melting points, gel strength, and viscosity.

In certain aspects, the present invention discloses recombinant gelatins of higher molecular weights than gelatins available from conventional animal sources. In one aspect, the present invention discloses recombinant gelatin of high molecular weight produced by expression of an altered collagen construct comprising multiple copies of the collagenous domain of one or more collagen types. In another aspect, high molecular weight recombinant gelatin is produced by crosslinking recombinant gelatin polypeptides. The present invention discloses methods for producing large molecular weight recombinant gelatins by using crosslinking agents known in the art to crosslink gelatin polypeptides.

The present invention discloses methods for manipulating the molecular weight, the level of hydroxylation, and the degree of cross-linking of recombinant gelatins as desired to allow for creation of recombinant gelatins having various specific and defined Bloom strengths, as well as recombinant gelatins having various specific and defined viscosities.

As described previously, recombinant gelatins can be produced directly through expression of engineered constructs or can be produced through processing of recombinant collagens. Collagen sequences from which recombinant collagens or recombinant gelatins can be derived include animal collagens and, in preferred embodiments, human collagens. Nucleic acid sequences encoding collagens have been generally described in the art. (See, e.g., Fuller and Boedtker (1981) Biochemistry 20:996-1006; Sandell et al. (1984) J Biol Chem 259:7826-34; Kohno et al. (1984) J Biol Chem 259:13668-13673; French et al. (1985) Gene 39:311-312; Metsaranta et al. (1991) J Biol Chem 266:16862-16869; Metsaranta et al. (1991) Biochim Biophys Acta 1089:241-243; Wood et al. (1987) Gene 61:225-230; Glumoff et al. (1994) Biochim Biophys Acta 1217:41-48; Shirai et al. (1998) Matrix Biology 17:85-88; Tromp et al. (1988) Biochem J 253:919-912; Kuivaniemi et al. (1988) Biochem J 252:633-640; Ala-Kokko et al. (1989) Biochem J 260:509-516; International Publication No. WO 01/34647.)

Recombinant gelatins produced directly from expression of specifically engineered constructs can exist as gelatins of single and uniform molecular species, or as gelatins containing pre-defined mixtures - in specified percentages, if desired - of more than one molecular species of recombinant gelatin. Recombinant gelatins derived from recombinant collagens are gelatins having molecular weight ranges more discrete than those of extracted gelatins, and have additional distinguishing properties, such as, e.g., derivation from single or from multiple specified collagen types.

The invention discloses single molecular species recombinant gelatins useful in the manufacture of gelatin capsules. Single molecular species recombinant gelatins are gelatins comprised of individual gelatin polypeptides having shared characteristics, e.g., comprising gelatin polypeptides having the same amino acid sequence and the same molecular weight. In preferred embodiments, single molecular species recombinant gelatins are produced directly from expression of specifically engineered DNA expression constructs using recombinant expression techniques known in the art. (See, e.g., International Publication No. WO 01/34646.)

In certain embodiments, the invention discloses compositions useful in capsule manufacture and having a gelatin component, wherein the gelatin component of such compositions consists of a single molecular species recombinant gelatin. In other embodiments, the gelatin component of such compositions further comprises a second single molecular species recombinant gelatin. Embodiments comprising multiple single molecular species recombinant gelatins are also contemplated. In compositions comprising more than one single molecular species recombinant gelatins, the different single molecular species recombinant gelatins can be present in specified percentages/ratios, or in unspecified ratios, to each other.

One aspect of gelatin critical to the manufacture of gelatin capsules is the stability of gelatin compositions throughout the preparation, manufacture, and storage of hard and soft gelatin capsules. Stability of a gelatin composition refers to the ability of a gelatin composition to retain various properties, such as, for example, viscosity, important to the manufacture of capsules over time. Thermal stability refers to the ability of a gelatin composition to retain various properties important to the manufacture of capsules over time and through variations in temperature. In the manufacture of gelatin capsules, solutions of gelatin are heated to temperatures ranging from 45-60°C, often for extended periods of time. Solutions of animal-derived gelatins maintained under such conditions are not thermally stable and are subject to thermal hydrolysis or enzymatic degradation, affecting many of the useful properties in capsule manufacture, including Bloom strength and viscosity. The extent of thermal hydrolysis is a function of time and temperature. For example, thermal degradation of animal-derived gelatins during capsule manufacture leads to a reduction in viscosity of the gelatin solution, thereby affecting the consistency and performance of the capsule manufacturing process.

In specific embodiments, the recombinant gelatins of the present invention can be used in the manufacture of gelatin capsules, including hard shell or hard gelatin capsules, typically produced from gelatin solutions, and soft shell or soft gelatin capsules, typically made from gelatin films. In specific embodiments of the present invention, a hard gelatin capsule comprising recombinant gelatin and a soft gelatin capsule comprising recombinant gelatin are disclosed as are methods for manufacturing these capsules. A selected gelatin can begin to melt at mouth temperature, easing swallowing, and become liquid at internal body temperature, such as within the stomach. In one embodiment, the present invention discloses recombinant gelatins with the dissolution rates of commercially available capsules and coatings. In another embodiment, the present invention discloses recombinant gelatins with improved resiliency, appropriate for use in capsules.

In certain applications, such as the manufacture of gelatin capsules, the brittleness and hardness developed by gelatin over time is an important parameter that can limit the shelf-life and usefulness of currently available animal-source/extracted gelatins. The ability to maintain viscosity over time is an especially valuable asset to manufacturers of gelatin-containing products, who must currently buy gelatin in sizable lots in order to maintain consistency of manufactured products. Furthermore, some manufacturing processes, such as the manufacture of hard gelatin capsules, currently require a blend of gelatin types, e.g., of type A and type B gelatins, in order to produce a material with the desired properties, as the use of type B gelatin alone results, for example, in a hard gelatin capsule that is too brittle for manufacture and use.

The present gelatin can also be used in encapsulation, including microencapsulation, of various pharmaceutical, nutritional, and nutraceutical products. Gelatin is also used in microencapsulation of various flavors, colors, and other additives; vitamins; and food supplements, including nutritional and diet supplements, and fat substitutes. In ingested products, it might be desirable to provide recombinant gelatin having stability against degradation in the acidic environment of the stomach, gut, etc.

Gelatin extracted from animal sources is a mixture of heterogeneous proteins, containing polypeptide fragments derived from more than one type of collagen. The present invention discloses recombinant gelatin derived from one type of collagen, free of any other type of collagen. In certain aspects, the present invention discloses single molecular species recombinant gelatin suitable for preparation of a pharmaceutical capsule.

The present invention discloses the use of the present recombinant gelatins in manufacture of capsules, films, etc., prepared for consumers with Kosher, Halal, vegetarian, or other diets that restrict the ingestion of products, including pharmaceutical products, containing specific animal-source materials.

The following examples explain the invention in more detail: The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention.

### EXAMPLES

Unless otherwise stated, the following materials and methods were used in the examples of the present invention.

### Example 1. Recombinant Gelatins

Various recombinant gelatins were produced, either by direct expression of specific fragments of human α1 type I collagen, or by deriving recombinant gelatin from recombinant human collagen type I (homotrimer of a 1 type I collagen). Methods for producing recombinant gelatins by either method are known in the art. (See, e.g., International Publication No. WO 01/34646.) Single molecular species gelatins were produced directly by expression of engineered DNA expression constructs containing polynucleotide sequences encoding segments of human α1 type I collagen chain, and include a 50 kD single molecular species gelatin (amino acid residue 531 to residue 1030 of human α1(I) collagen) (SEQ ID NO:1), a 65 kD single molecular species gelatin (amino acid residue 531 to residue 1192 of human α1(I) collagen) (SEQ ID NO:3), and a 100 kD single molecular species gelatin (amino acid residue 179 to residue 1192 of human α1(I) collagen, wherein Arg220 and Arg304 have been replaced with proline residues) (SEQ ID NO:6). Recombinant human gelatins were also obtained by thermal denaturation of fully hydroxylated, 65% hydroxylated, and non-hydroxylated recombinant human collagen α1 type I homotrimers.

### Example 2. Gel Strength (Bloom) of Recombinant Gelatins

Bloom values (gel strength) of various recombinant gelatins, including various single molecular species gelatins, prepared as described above in Example 1 were determined by conventional methods. Briefly, a 6.67% solution (w/v) of each gelatin material was prepared and maintained at a constant temperature (10°C) over 17 hours. A standard Texture Analyzer was used to measure the weight in grams required to depress a standard 0.5-inch diameter AOAC (Association of Official Agricultural Chemists) plunger 4 millimeters into the gel. The weight in grams required for depression of the plunger corresponds to the Bloom value. (See, e.g., United States Pharmacopoeia and Official Methods of Analysis of AOAC International, 17^{th} edition, Volume II.) Results of Bloom value determinations for various gelatin materials are shown in Table 1 below.

Bloom values for recombinant gelatin derived from non-hydroxylated recombinant collagen and for single molecular species 100 kD gelatin were similar to that of Sigma Type-B gelatin, representative of a commercial gelatin used in capsule manufacture. Recombinant gelatin obtained from fully hydroxylated recombinant collagen homotrimers had higher Bloom values than recombinant gelatin obtained from partially hydroxylated (e.g., 65%) or non-hydroxylated recombinant collagen homotrimers. These data showed that gel strength of recombinant gelatin is related to the degree of hydroxylation, and that modifying the degree of hydroxylation of recombinant collagens from which the recombinant gelatins were obtained resulted in recombinant gelatins having specific and defined Bloom values. Therefore, the present invention provides recombinant gelatins and methods for producing recombinant gelatins having defined or specific gel strength useful in the manufacture of hard and soft capsules.

Single molecular species gelatin obtained from expression of an engineered expression construct had Bloom values correlating with the molecular weight (e,g., amino acid sequence length) of the gelatin polypeptide. Specifically, a 50 kD single molecular species gelatin (SEQ ID NO:1) had a Bloom strength of 10 g; a 65 kD single molecular species gelatin (SEQ ID NO:3) had a Bloom strength of 145 g; and a 100 kD single molecular species gelatin (SEQ ID NO:6) had a Bloom strength of 225 g. (See Table 1.) This data indicated that gel strength of single molecular species gelatin is related to the molecular weight (e.g., amino acid sequence length) of the gelatin polypeptide. Therefore, the present invention provides methods for producing and compositions containing single molecular species recombinant gelatins having defined or specific gel strength useful in the manufacture of hard and soft capsules.

**TABLE 1**

| **Gelatin** | **Bloom Strength** |
|---|---|
| 100 kD single molecular species gelatin | 225 g |
| 65 kD single molecular species gelatin | 145 g |
| 50 kD single molecular species gelatin | 10 g |
| Recombinant gelatin derived from non-hydroxylated recombinant human collagen type I homotrimer | 200 g |
| Recombinant gelatin derived from 65% hydroxylated recombinant human collagen type I homotrimer | 290 g |
| Recombinant gelatin derived from fully hydroxylated recombinant human collagen type I homotrimer | 400 g |
| Commercial gelatin (Sigma Type B) | 225 g |

### Example 3. Viscosity of Various Gelatins

Viscosity of gelatin solutions is a critical parameter in the manufacture of hard and soft gel capsules. Viscosity values at various temperatures of 6.67% solutions (w/v) of various gelatins prepared as described above in Example 1 were determined. Results of viscosity values, in centipoises (cP) are shown in Table 2 below.

**TABLE 2**

| **Gelatin** | **30°C** | **40°C** | **50°C** | **60°C** |
|---|---|---|---|---|
| 100 kD single molecular species gelatin | 6.6 | 5.3 | 4.4 | 3.7 |
| 65 kD single molecular species gelatin | 4.8 | 3.7 | 3.1 | 2.6 |
| 50 kD single molecular species gelatin | 3.8 | 3.1 | 2.6 | 2.1 |
| Recombinant human gelatin derived from non-hydroxylated recombinant human collagen type I homotrimer | 7.0 | 5.3 | 4.3 | 3.6 |
| Recombinant human gelatin derived from 65% hydroxylated recombinant human collagen type I homotrimer | | 6.7 | 4.7 | 4.1 |
| Recombinant human gelatin derived from fully hydroxylated recombinant human collagen type I homotrimer | | 7.0 | 5.3 | 4.4 |
| Commercial gelatin (Sigma Type B) | | 5.8 | 4.7 | 4.0 |

As shown in Table 2 above, recombinant gelatin obtained from fully hydroxylated recombinant collagen homotrimers had higher viscosity values than recombinant gelatin obtained from partially hydroxylated (i.e., 65%) or non-hydroxylated recombinant collagen homotrimers. These results showed that viscosity of a gelatin solution is related to the degree of hydroxylation, and that modifying the degree of hydroxylation of recombinant collagens from which recombinant gelatins are obtained resulted in recombinant gelatins having specific and defined viscosity.

Single molecular species gelatins had viscosity values correlating with the molecular weight (e.g., amino acid sequence length) of the gelatin polypeptide. (See Table 2.) The present invention provides methods for producing recombinant gelatins and single molecular species gelatins having defined or specific viscosity values useful in the manufacture of hard and soft gel capsules. The present invention also provides compositions containing recombinant gelatins and single molecular species gelatins having defined or specific viscosity values useful in the manufacture of hard and soft gel capsules.

### Example 4. Capsule formation

A 100 kD single molecular species gelatin (SEQ ID NO:6) described above in Example 1 was molded to form a gelatin capsule as follows. Three grams of freeze-dried 100 kD single molecular species gelatin (SEQ ID NO:6) was dissolved in 6 ml of water at 60-65°C. The tip of a 0.6 cm x 20 cm steel rod was dipped into the gelatin solution and rotated several times to evenly coat the tip of the rod with the gelatin solution. The steel rod was removed from the gelatin solution and spun while a current of cold air was blown onto the tip of the rod until the gelatin solidified. The gelatin capsule was then removed from the steel rod by a gentle push.

### Example 5: Thermal stability and stable viscosity

Thermal stability of the gelatins of the present invention was compared to that of animal-derived gelatins as follows. Two grams of either 100 kD single molecular species gelatin (SEQ ID NO:6) or various animal-derived gelatins (Type A gelatin, 175 Bloom, from porcine skin; Type A gelatin, 300 Bloom, from porcine skin; and Type B gelatin, 225 Bloom, from bovine skin; all from Sigma Chemical Co.) were dissolved in 10 ml of water at 60°C (resulting is a 20% gelatin (w/v) solution). The viscosity of these 20% gelatin solutions was determined at 60°C using a Brookfield viscometer. Each gelatin solution was then incubated at 60°C for 17 hours, after which viscosity was again measured as described above. Viscosity values are presented in centipoises (cP). Percentage changes in viscosity measurements over time were determined. Results from these experiments are shown below in Table 3.

**TABLE 3**

| Condition | 100 kD (SEQ ID NO:6) | Type A Gelatin (175 Bloom) | Type A Gelatin (300 Bloom) | Type B Gelatin (225 Bloom) |
|---|---|---|---|---|
| 20% solution @ 60°C | 42.5 cP | 35.3 cP | 102.5 cP | 41.1 cP |
| 20% solution incubated 17 hr @ 60°C | 41.0 cP | 27.7 cP | 71.0 cP | 31.2 cP |
| Percent Viscosity Change | 3.5% | 21.5% | 30.7% | 24.1% |

Table 3 shows the results of viscosity measurements of various 20% gelatin solutions maintained at 60°C for 0 or 17 hours. As shown in Table 3, a 20% solution of recombinant human gelatin had a viscosity of 42.5 cP at 60°C. Following a 17-hour incubation at 60°C, the viscosity of the 20% solution of recombinant human gelatin was 41.0 cP. The change in viscosity of the recombinant human gelatin solution following a 17-hour incubation at 60°C corresponded to a 3.5% change (i.e., decrease) in viscosity. Solutions containing gelatin derived from animal sources showed a much greater decrease and overall change in viscosity following a 17-hour incubation at 60°C than recombinant human gelatin solution. Specifically, the percent decrease in viscosity for 20% solutions of Type A gelatin (175 Bloom), Type A gelatin (300 Bloom), and Type B gelatin (225 Bloom) following a 17-hour incubation at 60°C was 21.5%, 30.7%, and 24.1%, respectively. (See Table 3.)

These results demonstrated that single molecular species gelatins of the present invention are thermally stable. Additionally, these results showed that single molecular species gelatins of the present invention are more thermally stable than animal-derived gelatin, and therefore provide benefit for capsule manufacture. These results also showed that a 20% solution of single molecular species gelatin maintained viscosity following a 17-hour incubation at 60°C, indicating that single molecular species gelatins of the present invention have viscosities which are stable or thermally stable, e.g., which remain constant over a set period of time at a set temperature.

### Example 6: Mechanical properties of recombinant gelatin for capsules

The mechanical properties (force at break and elongation at break (elasticity)) of a gelatin film prepared with a single molecular species recombinant gelatin (100 kD, SEQ ID NO:6) or prepared with animal-derived gelatin were determined as follows. The gel mass of the recombinant human gelatin solution used for preparation of the gelatin film had a viscosity of 5,751 cP prior to casting a film. A 100 kD single molecular species recombinant gelatin film of 0.0030 inches in thickness was cast at room temperature onto a smooth surface. After a 30 minute setting time, the film was cut into strips 10 cm long and 2 cm wide. The mechanical properties of the gelatin strips were then tested using a Texture Analyzer by pulling the specimen at a speed of 10 mm per second until the gelatin strip breaks. The force and distance at break were measured and recorded as force at break in Newton (N) and as % of elongation at break, respectively.

The results on the force at break and elongation at break for a 100 kD single molecular species gelatin and for animal-derived gelatin are shown in Table 4.

**TABLE 4**

| Mechanical Property | 100 kD gelatin (SEQ ID NO:6) | Animal-derived gelatin |
|---|---|---|
| Force at break (N) | 11.6± 1.0 | 13 |
| Elongation at break (%) | 329.2 ± 24.2 | < 300 |

As shown in Table 4, the gelatin film prepared with 100 kD single molecular species recombinant gelatin required a force at break slightly weaker than but comparable to that of the gelatin file prepared with animal-derived gelatin. The percent elongation at break, however, was higher for the gelatin film prepared with a 100 kD single molecular species recombinant gelatin, indicative of the higher elasticity of the single molecular species recombinant gelatin film.

## Claims

1. A recombinant gelatin, wherein the recombinant gelatin is selected from the group consisting of SEQ ID NOs: 5, 6, 7, 8, 9, 10, 11, 12 and 13 and has a specific Bloom or gel strength useful in the manufacture of soft and hard gelatin capsules.

2. The recombinant gelatin according to claim 1, wherein the recombinant gelatin is SEQ ID NO: 6.

3. The recombinant gelatin according to claim 1 or claim 2, wherein the recombinant gelatin has a viscosity of 6.6, 5.3, 4.4 or 3.7 cP at 30°C, 40°C, 50°C or 60°C respectively, in a 6.67% (w/v) solution.

4. The recombinant gelatin according to any one of the preceding claims, wherein the recombinant gelatin has a Bloom strength of 225g.

5. A gelatin capsule comprising recombinant gelatin according to any of claims 1 to 4.

6. The capsule according to claim 5, wherein the capsule further comprises a capsule manufacturing agent.

7. A method for manufacturing a gelatin capsule, the method comprising providing a mixture comprising a recombinant gelatin as defined in any one of claims 1-4 and a capsule manufacturing agent, and forming a gelatin capsule from the mixture.

8. The capsule according to claim 6 or the method according to claim 7, wherein the capsule manufacturing agent is selected from the group consisting of a plasticizer, a coloring agent, an opacifying agent, a flavoring, a sugar, an acid and a medicament.

9. The capsule or method according to claim 8, wherein the capsule manufacturing agent is a plasticizer.

## Patentansprüche

1. Rekombinante Gelatine, wobei die rekombinante Gelatine ausgewählt ist aus der Gruppe, bestehend aus SEQ ID NOs: 5, 6, 7, 8, 9, 10, 11, 12 und 13, und eine spezifische Bloom-Stärke oder Gelstärke aufweist, die bei der Herstellung von Weich- und Hartgelatinekapseln nützlich ist.

2. Rekombinante Gelatine nach Anspruch 1, wobei die rekombinante Gelatine SEQ ID NO: 6 ist.

3. Rekombinante Gelatine nach Anspruch 1 oder 2, wobei die rekombinante Gelatine eine Viskosität von 6,6, 5,3, 4,4 oder 3,7 cP bei 30° C, 40° C, 50° C bzw. 60° C in einer 6,67 %igen (Gew/Vol) Lösung aufweist.

4. Rekombinante Gelatine nach einem der vorangehenden Ansprüche, wobei die rekombinante Gelatine eine Bloom-Stärke von 225 g aufweist.

5. Gelatinekapsel, die rekombinante Gelatine nach einem der Ansprüche 1 bis 4 umfasst.

6. Kapsel nach Anspruch 5, wobei die Kapsel weiterhin ein Mittel zum Herstellen von Kapseln umfasst.

7. Verfahren zum Herstellen einer Gelatinekapsel, wobei das Verfahren ein Verfügbarmachen einer Mischung, die eine rekombinante Gelatine, wie in einem der Ansprüche 1 bis 4 definiert, und ein Mittel zum Herstellen von Kapseln umfasst, und ein Bilden einer Gelatinekapsel aus der Mischung umfasst.

8. Kapsel nach Anspruch 6 oder Verfahren nach Anspruch 7, wobei das Mittel zum Herstellen von Kapseln ausgewählt ist aus der Gruppe, bestehend aus einem Weichmacher, einem Farbstoff, einem Trübungsmittel, einem Geschmacksstoff, einem Zucker, einer Säure und einem Medikament.

9. Kapsel oder Verfahren nach Anspruch 8, wobei das Mittel zum Herstellen von Kapseln ein Weichmacher ist.

## Revendications

1. Gélatine recombinante, ladite gélatine recombinante étant choisie dans le groupe consistant en les SEQ ID NO: 5, 6, 7, 8, 9, 10, 11, 12 et 13, et présentant une résistance Bloom ou de gel spécifique, utile dans la production de capsules de gélatine molle et de gélules.

2. Gélatine recombinante suivant la revendication 1, ladite gélatine recombinante ayant la SEQ ID NO:6.

3. Gélatine recombinante suivant la revendication 1 ou la revendication 2, ladite gélatine recombinante ayant une viscosité de 6,6, 5,3, 4,4 ou 3,7 cP à 30°C, 40°C, 50°C ou 60°C, respectivement, dans une solution à 6,67 % (en poids/volume).

4. Gélatine recombinante suivant l'une quelconque des revendications précédentes, ladite gélatine recombinante ayant une résistance Bloom de 225 g.

5. Capsule de gélatine comprenant la gélatine recombinante suivant l'une quelconque des revendications 1 à 4.

6. Capsule suivant la revendication 5, ladite capsule comprenant en outre un agent de production de capsules.

7. Procédé pour la production d'une capsule de gélatine, le procédé comprenant les étapes consistant à fournir un mélange comprenant une gélatine recombinante telle que définie dans l'une quelconque des revendications 1 à 4 et un agent de production de capsules, et à former une capsule de gélatine à partir du mélange.

8. Capsule suivant la revendication 6 ou procédé suivant la revendication 7, dans lequel l'agent de production de capsules est choisi dans le groupe consistant en un plastifiant, un colorant, un opacifiant, un agent aromatisant, un sucre, un acide et un médicament.

9. Capsule ou procédé suivant la revendication 8, dans lequel l'agent de production de capsules est un plastifiant.
